# EUROPEAN PATENT SPECIFICATION

(11) **EP 0 679 634 B1**
(45) Date of publication and mention of the grant of the patent: **07.01.1998**
(21) Application number: 95106339.5
(22) Date of filing: 27.04.1995
(51) Int. Cl.: C07C 231/10, C07C 235/74, C07D 233/14, C07D 473/06

(54) **Method for producing a 3-oxo-propionic acid amide compound and substituted acetyl compound used therein**
Verfahren zur Herstellung eines 3-Oxo-propionsäureamides und substituierte Acetylverbindung dafür
Procédé de préparation d'une amide de l'acide 3-oxo-propionique et composé acétyle substitué par ce faire

(30) Priority: 27.04.1994 JP 110140/94; 12.10.1994 JP 271849/94; 27.04.1994 JP 110142/94
(43) Date of publication of application: 02.11.1995
(73) Proprietor: FUJI PHOTO FILM CO., LTD., Kanagawa-ken (JP)
(72) Inventor: Yamakawa, Katsuyoshi, Minami-Ashigara-shi, Kanagawa-ken (JP); Sato, Tadahisa, Minami-Ashigara-shi, Kanagawa-ken (JP); Hanaki, Koichi, Minami-Ashigara-shi, Kanagawa-ken (JP)
(74) Representative: Hansen, Bernd, Dr. Dipl.-Chem.

(56) References cited:
- EP-A- 0 416 684
- US-A- 4 022 620
- US-A- 4 286 053

## Description

The present invention relates to a novel method for producing a yellow coupler used in silver halide color photographic materials and a synthetic intermediate of the coupler.

Further, the present invention relates to a novel substituted acetyl compound used as a synthetic intermediate, for example, in the production of a yellow coupler used in recording materials, such as silver halide color photographic materials, and in the production of intermediates of medicines.

### BACKGROUND OF THE INVENTION

As yellow couplers used in color photographic materials, pivaloylacetanilides and benzoylacetanilides are mainly used, and many two-equivalent couplers having a substituted imidazolyl group in the coupling site are known (see, e.g., U.S. Patent Nos. 4,046,575, 4,049,458, 4,133,958, and 4,032,347).

The methods for synthesizing these generally comprise, as shown below, reacting a β-ketoester compound 1, as a key intermediate, with anilines, to obtain a β-ketoanilide compound 2, followed by halogenation (to obtain a compound 3) and a substitution reaction (to obtain a compound 4) (JP-B ("JP-B" means examined and published Japanese patent publication) Nos. 10739/1983, 45135/1981, and 44420/1981): 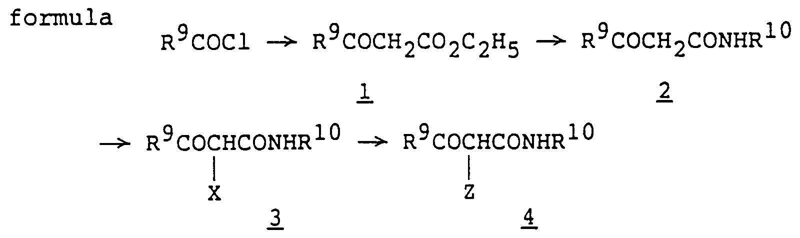 wherein R⁹ represents an alkyl group, an aryl group, or a nitrogen-containing heterocyclic group; R¹⁰ represents an aryl group; X represents a halogen atom; and Z represents a coupling releasing group, such as a nitrogen-containing heterocyclic group and a phenoxy group.

However, the reaction of a β-ketoester compound with anilines is carried out at a high temperature and takes a long time. Hence, in the method, such problems remain unsolved as that, anilines that have a thermally unstable substituent cannot be used in the reaction; in the step of introducing the coupling releasing group, partially reduction dehalogenation takes place; and the coupling releasing group is required to be used in excess. Therefore the method is desired to be improved (see, for example, U.S. Patent No. 4,230,851).

On the other hand, as a method for synthesizing a β-ketoanilide compound without a β-ketoester compound being used as a synthetic intermediate, for example, there are known a method (A) wherein methylketone is activated by converting it into an enamine, followed by heating together with an arylisocyanate prepared from anilines and phosgene, and a method (B) wherein methylketone is converted into lithium enolate by using a strong base called LDA (lithium diisopropylamide), followed by reaction with an arylisocyanate prepared from anilines and phosgene.

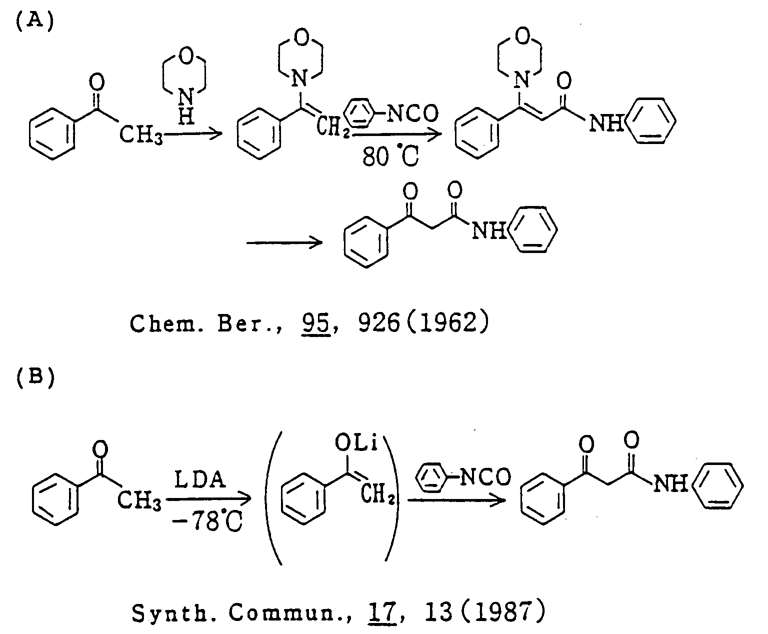

In particular, the latter method (B) is attractive because the reaction proceeds at a low temperature, but it is difficult to use a large amount of the strong base called LDA on an industrial scale, despite that LDA is a common laboratory reagent. Further, it is required that the arylisocyanto be prepared using phosgene, which is toxic, which is a problem because, for example, the facilities for the production are limited.

### SUMMARY OF THE INVENTION

A first object of the present invention is to provide a method for producing a yellow coupler used in silver halide color photographic materials and a synthetic intermediate of the coupler, easily under mild conditions without using a highly toxic compound, such as phosgene.

A second object of the present invention is to overcome the conventional difficulties concerning the above synthesis of yellow couplers by providing a novel synthetic intermediate useful in easily synthesizing a yellow coupler used in silver halide color photographic materials.

Other and further objects, features, and advantages of the invention will appear more fully from the following description.

### DETAILED DESCRIPTION OF THE INVENTION

The first object of the present invention has been attained by the following method:

That is, the present invention provides a method for producing a compound represented by the following formula (IV): 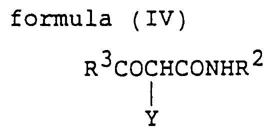 wherein R² represents an alkyl group, a cycloalkyl group, or an aryl group; R³ represents an alkyl group bonded through a secondary or tertiary carbon atom, a cycloalkyl group, a cyclic ether group, an aryl group, a group represented by the formula (III), an alkoxy group, or an N,N-di-substituted amino group, in which N,N-di-substituted amino group the substituents may bond together to form a nitrogen-containing heterocyclic ring together with the nitrogen atom; and Y represents a hydrogen atom or an electron-attracting group: 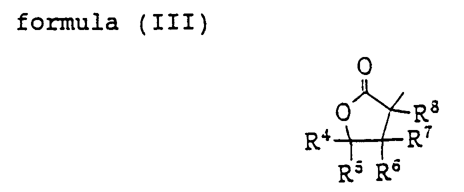 wherein R⁴, R⁵, R⁶, R⁷, and R⁸ each represent a hydrogen atom, an alkyl group, a benzyl group, an aryl group, an alkoxy group, an aryloxy group, an alkylthio group, or an arylthio group, comprising reacting a compound represented by the formula (I):

R¹OCONHR² formula (I)

wherein R¹ represents an aryl group and R² has the same meaning as that in formula (IV), with a compound represented by the formula (II):

R³COCH₂Y formula (II)

wherein R³ and Y each have the same meaning as those in formula (IV), in the presence of a base (hereinafter this method is referred to as first invention of the present invention).

The second object has been attained by the following substituted acetyl compound represented by the formula (IX):

R¹⁶ - COCH₂R¹⁷ formula (IX)

wherein R¹⁶ represents a tert-butyl group or a phenyl group having an alkoxy group with 1 to 18 carbon atoms in the paraposition, and wherein R¹⁷ represents a group represented by the formula (X) or the formula (XI): 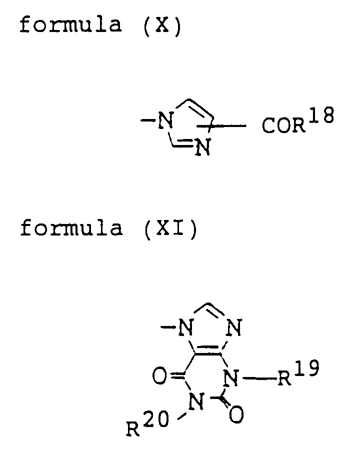 wherein R¹⁸ represents an alkoxy group having 1 to 18 carbon atoms or an anilino group, and R¹⁹ and R²⁰ each represent an alkyl group having 1 to 18 carbon atoms (hereinafter the compound stated in the above (1) is referred to as second invention of the present invention).

The substituted acetyl compound of the second invention is preferably
(2) a substituted acetyl compound as stated in the above (1), wherein in formula (IX) the 1-imidazolyl group represented by formula (X) has a group -COR¹⁸ in the 2-position and R¹⁸ represents an anilino group, or
(3) a substituted acetyl compound as stated in the above (1), wherein in formula (IX) the 1-imidazolyl group represented by formula (X) has a group -COR¹⁸ in the 4- or 5-position and R¹⁸ represents an alkoxy group having 1 to 18 carbon atoms.

In this specification, "the present invention" denotes both the above first and second inventions, unless otherwise specified.

The production method of the first invention of the present invention will now be described in detail.

First the compounds are described in detail.

R¹ represents an aryl group (preferably having a C-number of 6 to 26 (i.e., having 6 to 26 carbon atoms, the same being applied hereinafter), e.g., phenyl and naphthyl), which may be substituted. The substituent includes, for example, an alkyl group (preferably having a C-number of 1 to 18, e.g., methyl, ethyl, isopropyl, tert-butyl, and tert-octyl), an aryl group (preferably having a C-number of 6 to 26, e.g., phenyl and naphthyl), a halogen atom (e.g., fluorine, chlorine, bromine, and iodine), a cyano group, a nitro group, an alkylsulfonyl group (preferably having a C-number of 1 to 18, e.g., methanesulfonyl and butanesulfonyl), an arylsulfonyl group (preferably having a C-number of 6 to 26, e.g., benzenesulfonyl and p-toluenesulfonyl), an alkoxycarbonyl group (preferably having a C-number of 2 to 18, e.g., methoxycarbonyl and hexadecyloxycarbonyl), an alkoxy group (preferably having a C-number of 1 to 18, e.g., methoxy, ethoxy, and octadecyloxy), and an aryloxy group (preferably having a C-number of 6 to 26, e.g., phenoxy).

More preferably, R¹ represents an unsubstituted phenyl group or a substituted phenyl group whose substituent is an alkyl group having 1 to 18 carbon atoms, a chlorine atom, a bromine atom, or a nitro group. More preferably, R¹ represents a phenyl group, a p-methylphenyl (p-tolyl) group, an m-methylphenyl (m-tolyl) group, an o-methylphenyl (o-tolyl) group, a p-chlorophenyl group, an m-chlorophenyl group, an o-chlorophenyl group, a p-nitrophenyl group, an m-nitrophenyl group, or an o-nitrophenyl group. When the substituent is methyl group, chlorine atom, or a nitro group, R¹ may be made up of a mixture of o-, m-, and p-isomers.

R¹ is particularly preferably a phenyl group.

Preferably R² represents a straight-chain or branched-chain alkyl group (preferably having a C-number of 1 to 18, e.g., methyl, ethyl, and propyl), a cycloalkyl group (preferably having a C-number of 3 to 18, e.g., cyclohexyl and cyclopropyl), or an aryl group (preferably having a C-number of 6 to 26, e.g., phenyl and naphthyl).

When R² represents an alkyl group, the said alkyl group may be substituted by an alkoxy group having 1 to 18 carbon atoms (e.g., methoxy, dodecyloxy, hexadecyloxy, and octadecyloxy), or an aryloxy group having 6 to 26 carbon atoms (e.g., 2,4-di-tert-amylphenoxy and 2,4-di-tert-octylphenoxy). When R² represents an aryl group, the aryl group may be substituted by a variety of groups, and the substituent includes the substituents of R¹, i.e., an alkyl group (preferably having a C-number of 1 to 18, e.g., methyl, ethyl, isopropyl, (t)-butyl, and (t)-octyl), an aryl group (preferably having a C-number of 6 to 26, e.g., phenyl and naphthyl), a halogen atom (e.g., fluorine, chlorine, bromine, and iodine), a cyano group, a nitro group, an alkylsulfonyl group (preferably having a C-number of 1 to 18, e.g., methanesulfonyl and butanesulfonyl), an arylsulfonyl group (preferably having a C-number of 6 to 26, e.g., benzenesulfonyl and p-toluenesulfonyl), an alkoxycarbonyl group (preferably having a C-number of 2 to 18, e.g., methoxycarbonyl and hexadecyloxycarbonyl), an alkoxy group (preferably having a C-number of 1 to 18, e.g., methoxy, ethoxy, and octadecyloxy), and an aryloxy group (preferably having a C-number of 6 to 26, e.g., phenoxy), as well as an acylamino group (preferably having a C-number of 1 to 18, e.g., acetylamino, palmitoylamino, and 2-(2,4-di-tert-amylphenoxy)butanoylamino), an alkylsulfonamido group (preferably having a C-number of 1 to 18, e.g., methanesulfonamido, butanesulfonamido, and hexadecanesulfonamido), an arylsulfonamido group (preferably having a C-number of 6 to 26, e.g., 2-butoxy-5-tert-octylphenylsulfonamido), a ureido group (preferably having a C-number of 1 to 18, e.g., 3-(4-cyanophenyl)ureido), an alkylsulfamoyl group (preferably having a C-number of 1 to 18, e.g., N,N-dimethylsulfamoyl and N-methylsulfamoyl), an arylsulfamoyl group (preferably having a C-number of 6 to 26, e.g., 2-chlorophenylsulfamoyl and 2-chloro-5-hexadecylsulfamoylphenylsulfamoyl), an acylsulfamoyl group (preferably having a C-number of 2 to 18, e.g., acetylsulfamoyl and ethanoylsulfamoyl), an alkylcarbamoyl group (preferably having a C-number of 1 to 18, e.g., N-butylcarbamoyl and N,N-dioctylcarbamoyl), and an arylcarbamoyl group (preferably having a C-number of 6 to 26, e.g., 2-chloro-5-dodecyloxycarbonylphenylcarbamoyl).

Preferably R² represents a phenyl group optionally substituted by the above substituent. The position of the substituent on the phenyl group is not particularly restricted, but preferably the position is at least one of the 2-, 4-, and 5-positions.

In the first invention, preferably the compound represented by formula (I) is a compound wherein R¹ is a phenyl group, and R² is a phenyl group that may be substituted by a halogen atom, an alkoxycarbonyl group, an acylamino group, an alkylsulfamoyl group, an arylsulfamoyl group, an alkylsulfonamido group, an alkoxy group, a nitro group, a cyano group, or an acylsulfamoyl group; and more preferably R² is a phenyl group that may be substituted by a halogen atom, an alkoxycarbonyl group, an acylamino group, an arylsulfamoyl group, an alkylsulfonamido group, or an alkoxy group.

R³ represents an alkyl group bonded through a secondary or tertiary carbon (preferably having a C-number of 1 to 18, e.g., isopropyl, sec-butyl, tert-butyl, and 1,1,3,3-tetramethylbutyl), a cycloalkyl group (preferably having a C-number of 3 to 18, e.g., cyclopropyl, 1-methylcyclopropyl, 1-ethylcyclopropyl, 1-benzylcyclopropyl, cyclohexyl, and adamantyl), a cyclic ether group (preferably having a C-number of 2 to 18, e.g., 2,2,5-trimethyl-1,3-dioxane-5-yl), an aryl group (preferably having a C-number of 6 to 26, e.g., phenyl and naphthyl), a 4-butanolido group represented by formula (III), an alkoxy group (preferably having a C-number of 1 to 18 carbon atoms, e.g., methoxy, ethoxy, and hexadecyloxy), or an N,N-disubstituted amino group (preferably having a C-number of 2 to 26, e.g., N,N-dimethylamino, N-methyl-N-phenylamino, and N,N-diphenylamino). When the substituents in the disubstituted amino group are not bonded together, preferably the substituents each represent an alkyl group having a C-number of 1 to 18 or an aryl group having a C-number of 6 to 26.

When R³ represents an aryl group, the aryl group may have a variety of substituents, which are the substituents of the aryl group represented by R².

When R³ represents an N,N-disubstituted amino group, the substituents may bond together to form a nitrogen-containing heterocyclic ring together with the nitrogen atom. The nitrogen-containing heterocyclic ring is preferably a 5- to 6-membered ring, including pyrrolidine, piperidine, piperazine, morpholine, indoline, and tetrahydroquinoline.

More preferably R³ represents a tert-butyl group, a cyclopropyl group having an alkyl group with 1 to 8 carbon atoms in the 1-position, a 1-benzylcyclopropyl group, a 2,2,5-trimethyl-1,3-dioxane-5-yl group, a phenyl group having an alkoxy group with 1 to 20 carbon atoms in the paraposition, a group represented by formula (III), an alkoxy group having 1 to 20 carbon atoms, or an indoline-1-yl group.

Particularly preferably R³ represents a tert-butyl group, a phenyl group having an alkoxy group with 1 to 20 carbon atoms in the paraposition, a group represented by formula (III), or an indoline-1-yl group.

When R³ is a 4-butanolido group represented by formula (III), its substituents: R⁴, R⁵, R⁶, R⁷, and R⁸, each represent a hydrogen atom, an alkyl group (preferably having a C-number of 1 to 18, e.g., methyl, ethyl, and propyl), a benzyl group, an aryl group (preferably having a C-number of 6 to 26, e.g., phenyl and naphthyl), an alkoxy group (preferably having a C-number of 1 to 18, e.g., methoxy, ethoxy, and 2-ethylhexyloxy), an aryloxy group (preferably having a C-number of 6 to 26, e.g., phenoxy and naphthyloxy), an alkylthio group (preferably having a C-number of 1 to 18, e.g., methylthio and ethylthio), or an arylthio group (preferably having a C-number of 6 to 26, e.g., phenylthio and naphthylthio).

More preferably R⁴, R⁵, R⁶, R⁷, and R⁸ each represent a hydrogen atom, an alkyl group having 1 to 8 carbon atoms, a benzyl group, or an aryl group having 6 to 15 carbon atoms. Particularly preferably R⁴, R⁵, R⁶, and R⁷ each represent a hydrogen atom. Particularly preferably R⁸ represents an alkyl group having 1 to 8 carbon atoms or a benzyl group.

Y represents a hydrogen atom or an electron-attracting group. Herein the term "an electron-attracting group" means a substituent wherein the substituent constant σₘ defined by Hammett is a positive value. The σₘ values of typical substituents are shown in Kagakuno Ryoiki "Yakubutsu no kozokasseisokan", Special number 122, Nankodo, 1979. Of course, the electron-attracting group in the present invention is not limited to the substituents shown in the above book (e.g., see C. Harsch et al., J. Med. Chem., 16, 1207 (1973); and 20, 304 (1977) Chem. Rev. 91, 165 (1991)).

Preferably Y represents a hydrogen atom, a cyano group (σₘ = 0.56), an alkylcarbonyl group having 2 to 18 carbon atoms (e.g., acetyl (σₘ = 0.38), propanoyl, butanoyl, pivaloyl (σₘ = 0.27), and pentadecanoyl), an arylcarbonyl group (e.g., benzoyl (σₘ = 0.34)), a nitro group (σₘ = 0.71), an alkoxycarbonyl group having 2 to 18 carbon atoms (e.g., methoxycarbonyl (σₘ = 0.37), ethoxycarbonyl (σₘ = 0.37), butoxycarbonyl, tert-butoxycarbonyl, and hexadecyloxycarbonyl), a carbamoyl group, a substituted carbamoyl group having 2 to 18 carbon atoms (e.g., N-methylcarbamoyl (σₘ = 0.35), N,N-dimethylcarbamoyl, N,N-dioctylcarbamoyl, N-methyl-N-phenylcarbamoyl, morpholinocarbonyl, piperidinocarbonyl, pyrrolidinocarbonyl, and indolinocarbonyl), an alkylsulfonyl group having 1 to 18 carbon atoms (e.g., methanesulfonyl (σₘ = 0.60) and butanesulfonyl), an arylsulfonyl group having 6 to 20 carbon atoms (e.g., benzenesulfonyl (σₘ = 0.62) and p-toluenesulfonyl), an aryloxy group having 6 to 20 carbon atoms (e.g., phenoxy (σₘ = 0.25), naphthyloxy, 4-methoxycarbonylphenoxy, and 4-(4-benzyloxyphenylsulfonyl)phenoxy), or a heterocyclic group.

In the arylcarbonyl group, the arylsulfonyl group, and the aryloxy group, the aryl group is preferably a substituted or unsubstituted phenyl group, and the substituent includes those mentioned for the aryl group represented by R².

The heterocyclic group is preferably a group derived from a 5- to 7-membered compound containing at least one nitrogen atom, oxygen atom, or sulfur atom, with which a benzene ring or a 5- to 7-membered heterocyclic ring may be condensed. Preferably the heterocyclic group is a 5- to 7-membered compound having at least one nitrogen atom, and the atom bonded as a substituent is a nitrogen atom or a carbon atom. As the heterocyclic group bonded through a carbon atom, typical examples are an oxazolyl group, a 2-benzoxazolyl group (σₘ = 0.30), a 2-benzothiazolyl group (σₘ = 0.27), a 2-furyl group (σₘ = 0.06), a 2-thienyl group (σₘ = 0.09), a 2-imidazolyl group, a 2-benzimidazolyl group, a 3-pyrazolyl group, a 1,2,3-triazole-4-yl group, a 1,2,4-triazole-3-yl group, a 5-tetrazolyl group, and a 2-pyridyl group (σₘ = 0.33).

As the heterocyclic group bonded through a nitrogen atom, typical examples are an azole-type heterocyclic group, such as a 1-pyrrolyl group (σₘ = 0.47), a 1-indolyl group, a 1-pyrazolyl group, a 1-imidazolyl group, a 1-benzimidazolyl group, a theophylline-1-yl group, a 1,2,3-triazole-1-yl group, a benzotriazole-1-yl group, a benzotriazole-2-yl group, a 1,2,4-triazole-1-yl group, a 1,2,4-triazole-4-yl group, and a tetrazole-1-yl group; and an imido-type heterocyclic group represented, for example, by formula (V), (VI), or (VII). 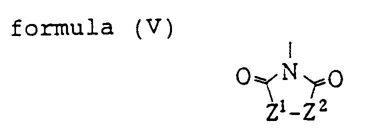 wherein Z¹ and Z² each represent an oxygen atom, a group represented by -N(R¹¹)-, or a group represented by -(R¹²)C(R¹³)-, wherein R¹¹ and R¹² each represent a hydrogen atom, an alkyl group, an benzyl group, or an aryl group, and R¹³ represents a hydrogen atom, an alkyl group, a benzyl group, an aryl group, or an alkoxy group. 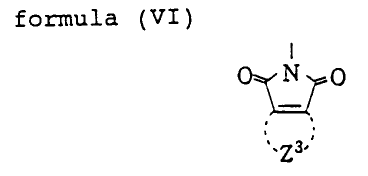 wherein Z³ represents a group of nonmetal atoms required to form a 5- to 7-membered ring. 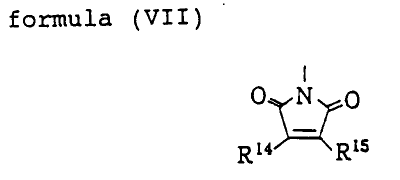 wherein R¹⁴ and R¹⁵ each represent a hydrogen atom, an alkyl group, or an aryl group.

In formula (V), when Z¹ represents an oxygen atom, preferably Z² is a group represented by -(R¹²)C(R¹³)-.

In formula (VI), the 5- to 7-membered ring formed by Z³ includes a benzene ring and a 5- to 7-membered unsaturated heterocyclic ring containing at least one nitrogen, oxygen, or sulfur atom (e.g., pyrrole, pyrazole, imidazole, 1,2,3-triazole, oxazole, thiazole, isooxazole, isothiazole, furan, thiophene, pyridine, pyrazine, pyrimidine, pyridazine, azepine, and oxepine), with preference given to a benzene ring.

The heterocyclic ring bonded through a nitrogen atom includes the imido-type heterocyclic rings represented by formulas (V) to (VII), as well as the 5-membered heterocyclic ring represented by formula (VIII). 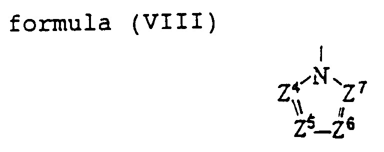 wherein Z⁴, Z⁵, Z⁶, and Z⁷ each represent a nitrogen atom or a substituted or unsubstituted methine group, and Z⁴ and Z⁵, Z⁵ and Z⁶, or Z⁶ and Z⁷ may bond together to form a 5- to 7-membered ring.

In formula (VIII), Z⁴, Z⁵, Z⁶, and Z⁷ each do not represent a nitrogen atom at the same time, and when Z⁴, Z⁵, Z⁶, or Z⁷ represents a substituted methine group, the substituent includes, for example, an alkyl group having 1 to 18 carbon atoms, an aryl group having 6 to 18 carbon atoms, a cyano group, a nitro group, an alkoxy group having 1 to 18 carbon atoms, an acylamino group having 1 to 18 carbon atoms, an alkylcarbamoyl group having 2 to 18 carbon atoms, an arylcarbamoyl group having 7 to 18 carbon atoms, and an alkoxycarbonyl group having 2 to 18 carbon atoms. When Z⁴ and Z⁵, Z⁵ and Z⁶, or Z⁶ and Z⁷ bond together to form a 5- to 7-membered ring, the ring includes those rings formed by Z³ in formula (VI).

Particularly preferably Y represents a hydrogen atom, a cyano group, an alkylcarbonyl group having 2 to 18 carbon atoms, an alkoxycarbonyl group having 2 to 18 carbon atoms, an aryloxy group having 6 to 20 carbon atoms, an imido-type 5-membered heterocyclic ring represented by formula (V), (VI), or (VII), or a 5-membered heterocyclic ring bonded through the nitrogen represented by formula (VIII).

Although the σₘ values of these heterocyclic rings are not all known, the σₘ value of a 1-pyrroline-2,5-dione is 0.34, and the σₘ values of other heterocyclic rings are supposed to be about the same.

More preferably Y represents a cyano group, an alkylcarbonyl group having 2 to 18 carbon atoms, an alkoxycarbonyl group having 2 to 18 carbon atoms, an aryloxy group having 6 to 20 carbon atoms, an imido-type 5-membered heterocyclic group represented by formula (V), (VI), or (VII), or a 5-membered heterocyclic group bonded through the nitrogen atom represented by formula (VIII).

Particularly preferably, when Y represents a cyano group, an alkylcarbonyl group having 2 to 18 carbon atoms, or an alkoxycarbonyl group having 2 to 18 carbon atoms, R³ represents an indoline-1-yl group.

Now the production method of the first invention will be described in detail.

The production method of the first invention is represented by the following reaction scheme:

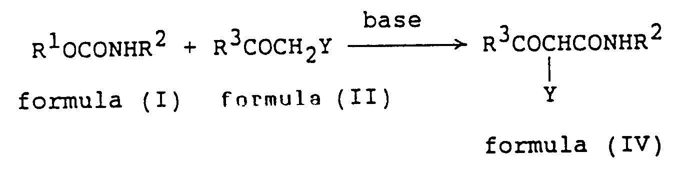

The synthesis of the starting compounds is first described.

The compound represented by formula (I) can be obtained easily by reaction of an amine, (R²NH₂), with a chlorocarbonate ester, (ClCO₂R¹). As the solvent that is often used in this reaction, an amide-type solvent, such as acetonitrile, N,N-dimethylformamide, and N,N-dimethylacetamide, and an ether-type solvent, such as ether, tetrahydrofuran (THF), and dioxane, can be mentioned.

As a base, a pyridine-type compound, such as pyridine, α-picoline, and 2,6-lutidine, and a tertiary amine compound, such as triethylamine, can be used. The reaction can be carried out without using a base but by heating, to remove the hydrochloric acid during the course.

The compound represented by formula (II) can be obtained, as desired, by reacting the corresponding chloroacetylated compound or bromoacetylated compound (ClCH₂COR³ or BrCH₂COR³), with a compound represented by Y-H, with the reaction molar ratio preferably being from 1 : 5 to 5 : 1, in the presence of a base. As the base, an organic base, for example, a pyridine-type compound, such as pyridine, α-picoline, and 2,6-lutidine, and a tertiary amine compound, such as DBU, DBN, DABCO, and triethylamine, as well as potassium carbonate, sodium carbonate, potassium tert-butoxide, sodium methoxide, sodium ethoxide, and the like, can be used. As the reaction solvent, the solvent that can be used for the synthesis of formula (I) can be used. The reaction temperature is preferably 0 to 140°C.

Now the production method will be described in detail.

The substituted or unsubstituted acetyl compound represented by formula (II) is used in an amount of 0.3 to 5 mol, preferably 0.5 to 2 mol, per mol of the urethanes represented by formula (I).

As the base, for example, LDA (lithium diisopropylamide), sodium hydride, potassium tert-butoxide, and DBU (1,8-diazabicyclo[5.4.0]-7-undecene) are used, with preference given to sodium hydride.

The amount of the base to be used is 0.9 to 5 mol, preferably 1 to 3 mol, per mol of the compound of formula (I).

As the reaction solvent, an ether-type solvent, such as ether, tetrahydrofuran, and dioxane, and an amide-type solvent, such as N,N-dimethylformamide, N,N-dimethylacetamide, N,N-dimethylimidazolidinone, and N-methylpyrrolidone, as well as an aprotic polar solvent, such as sulfolane, DMSO (dimethylsulfoxide), and HMPA (hexamethylphosphoric acid triamide), are preferably used.

The reaction temperature is -20 to 130°C, preferably -10 to 100°C, more preferably 0 to 60°C.

Specific examples of the compound represented by formula (I) and the compound represented by formula (IV) are shown below, but compound used in the first invention is not restricted to them.

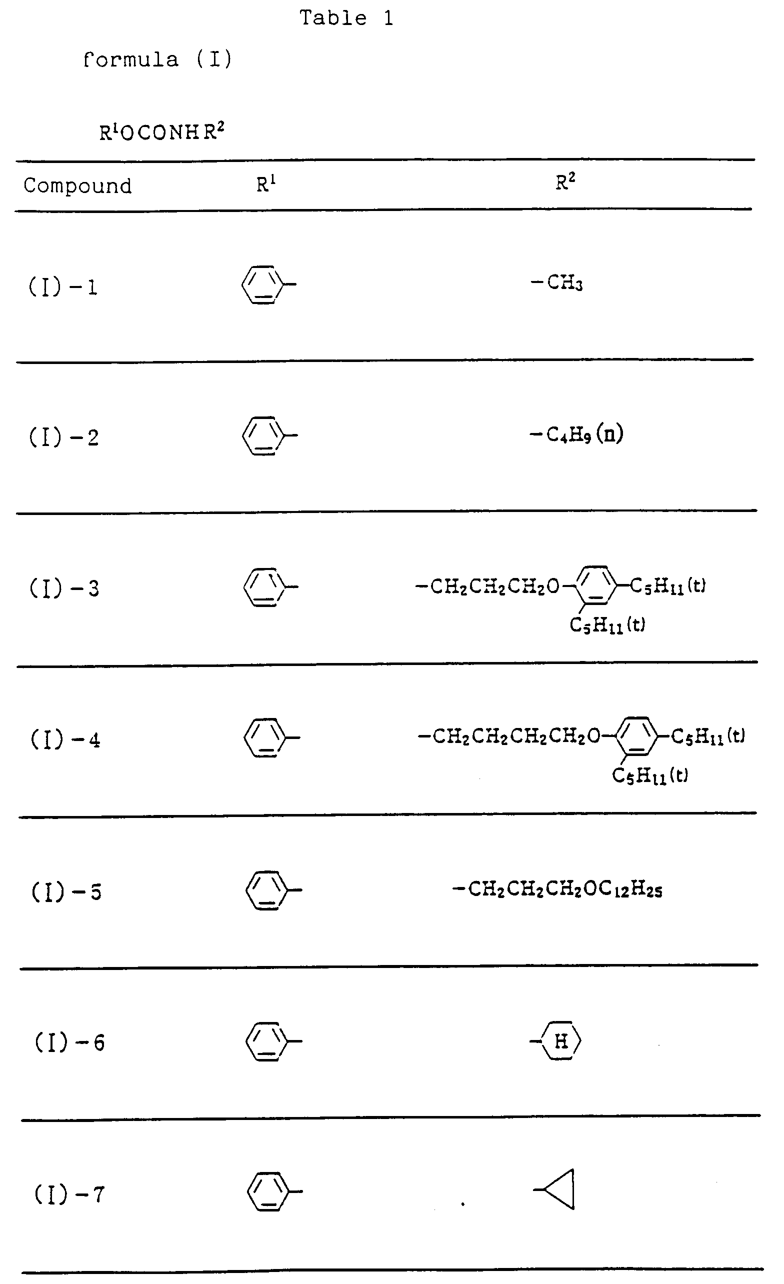

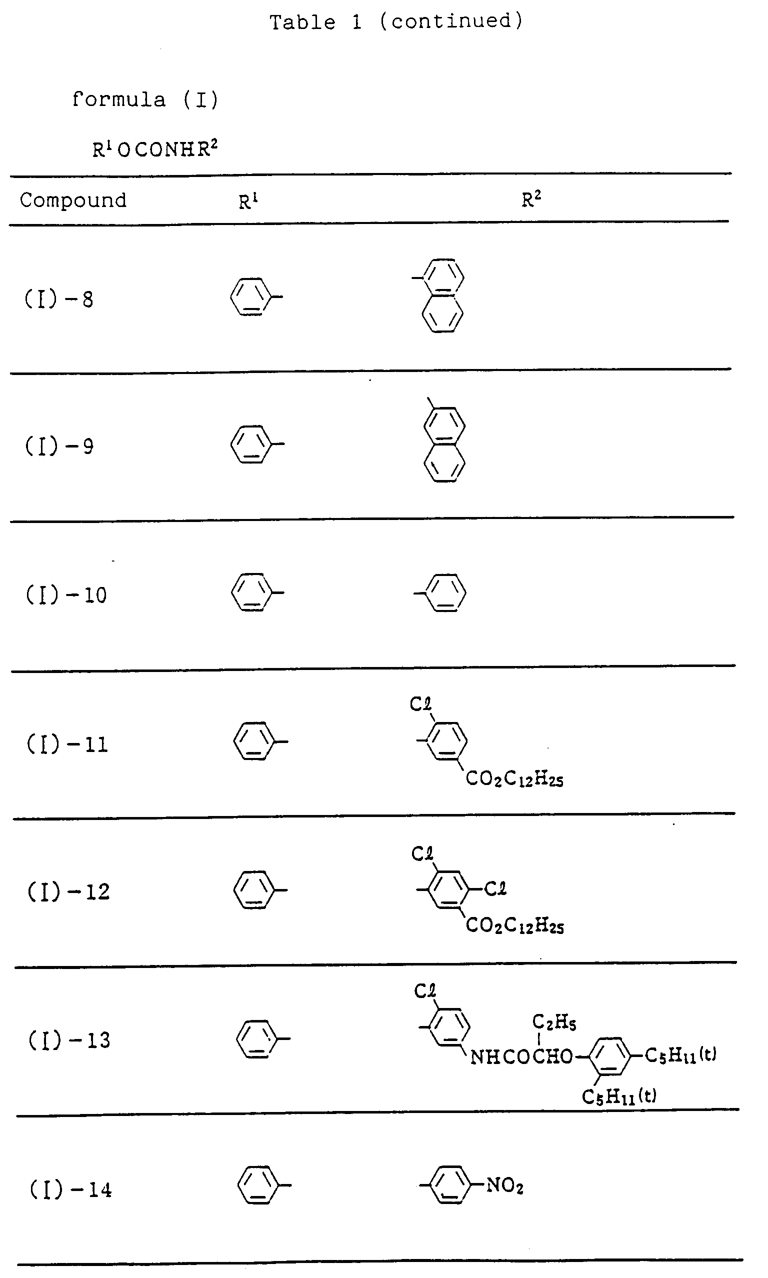

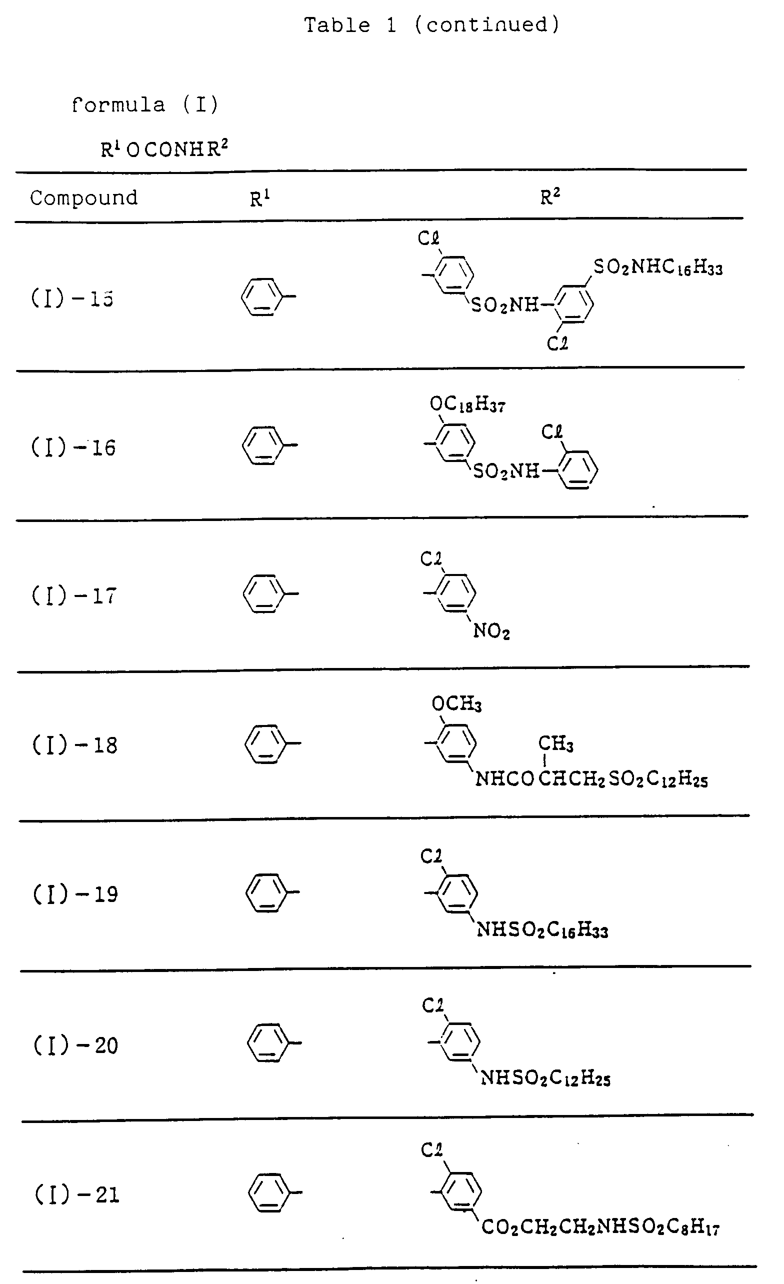

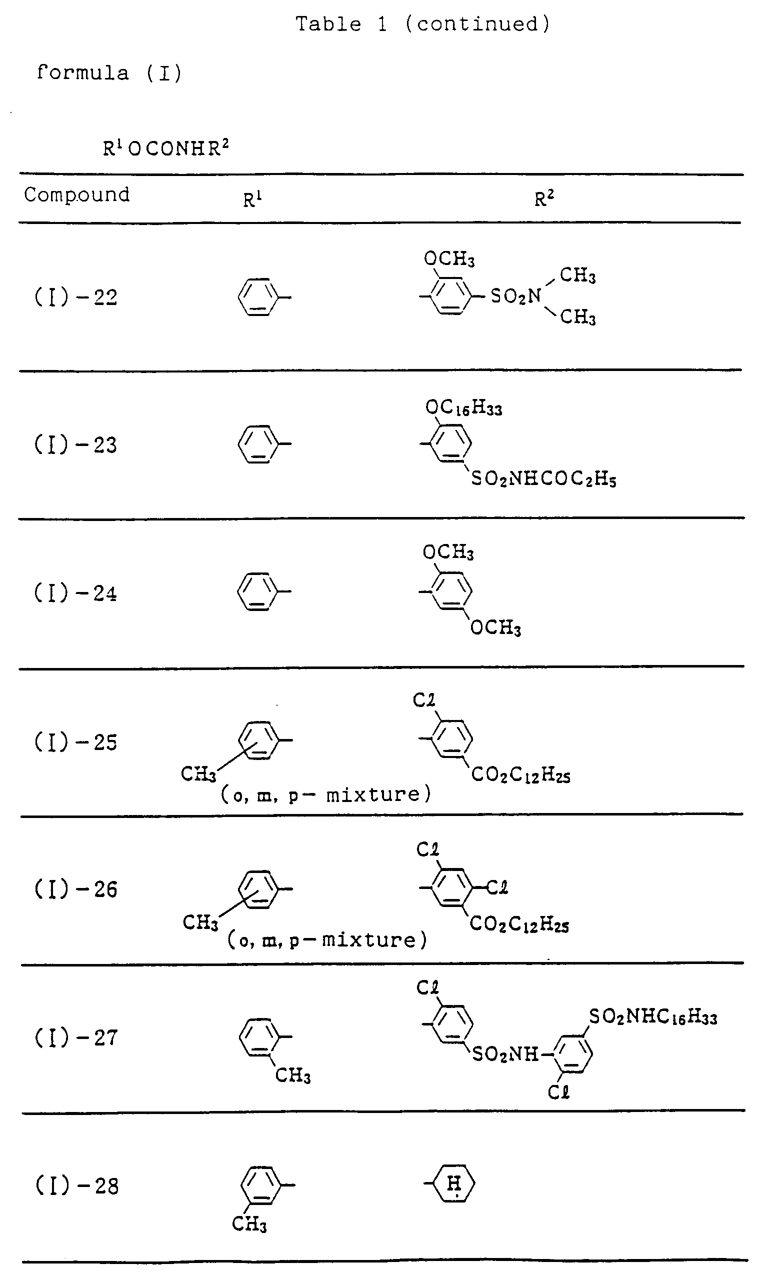

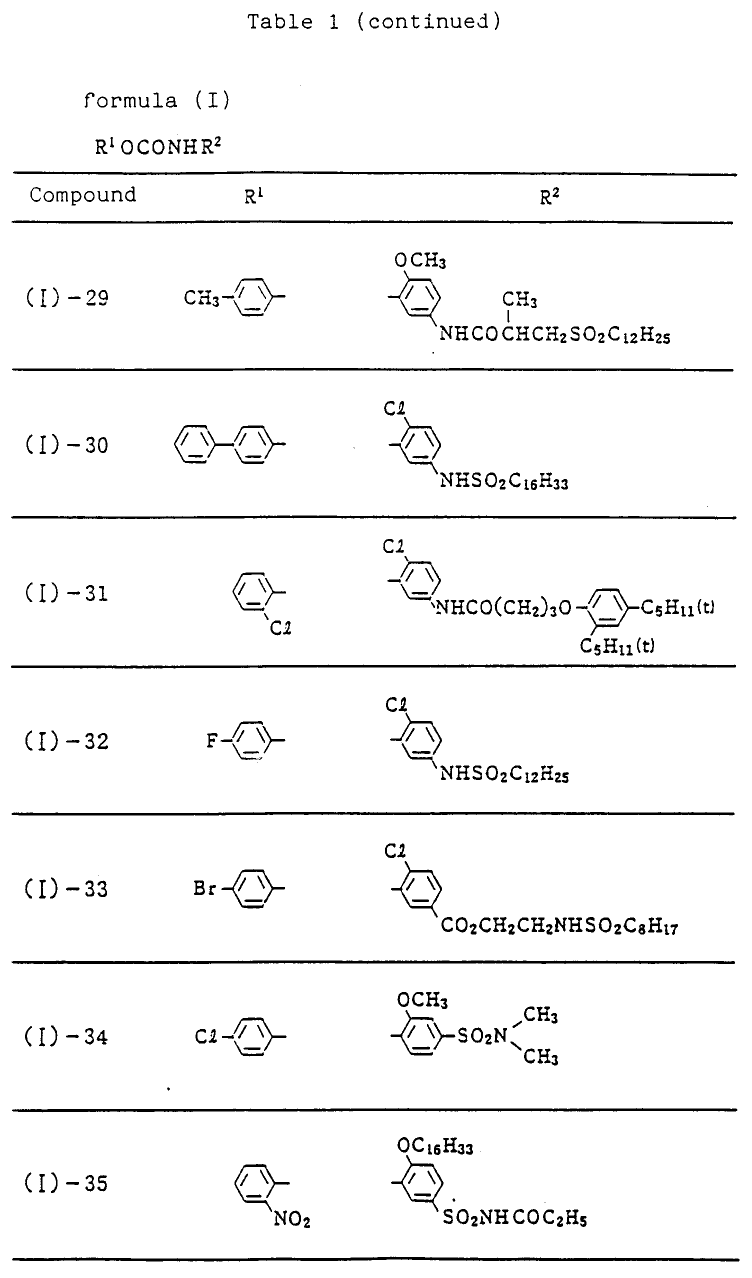

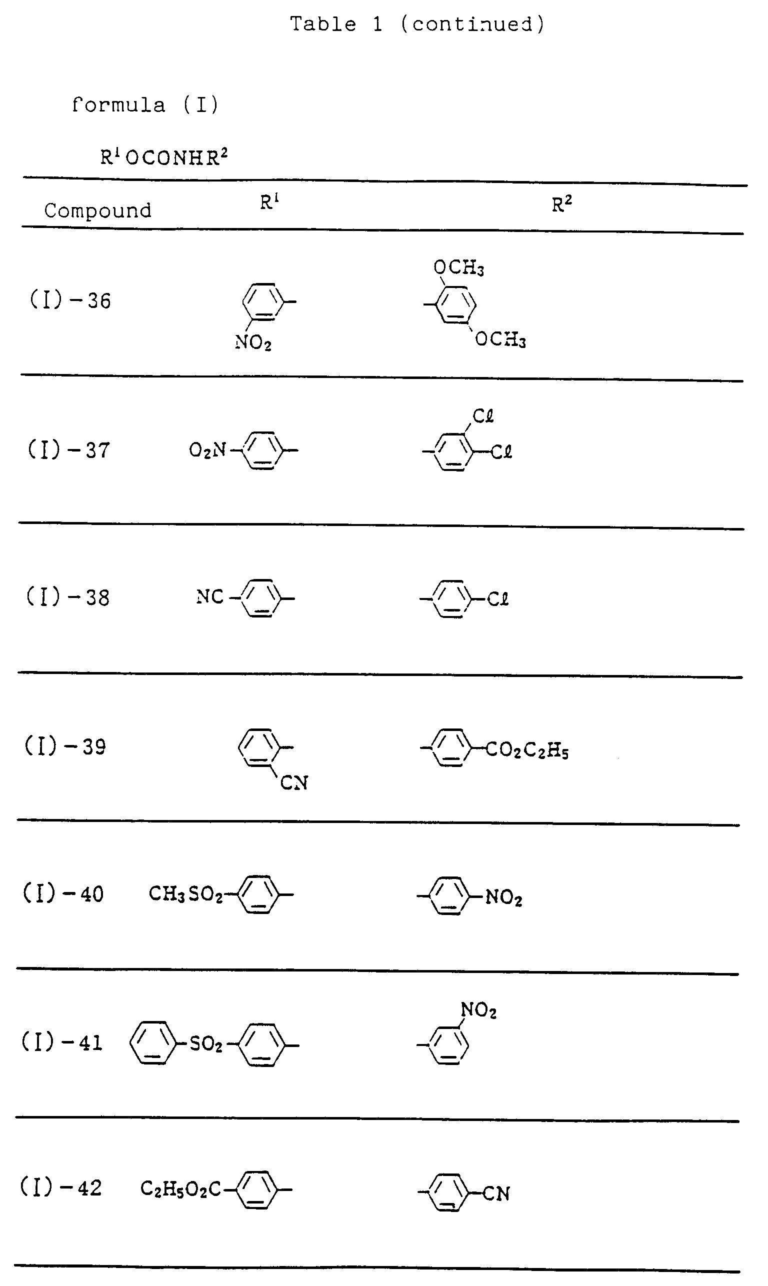

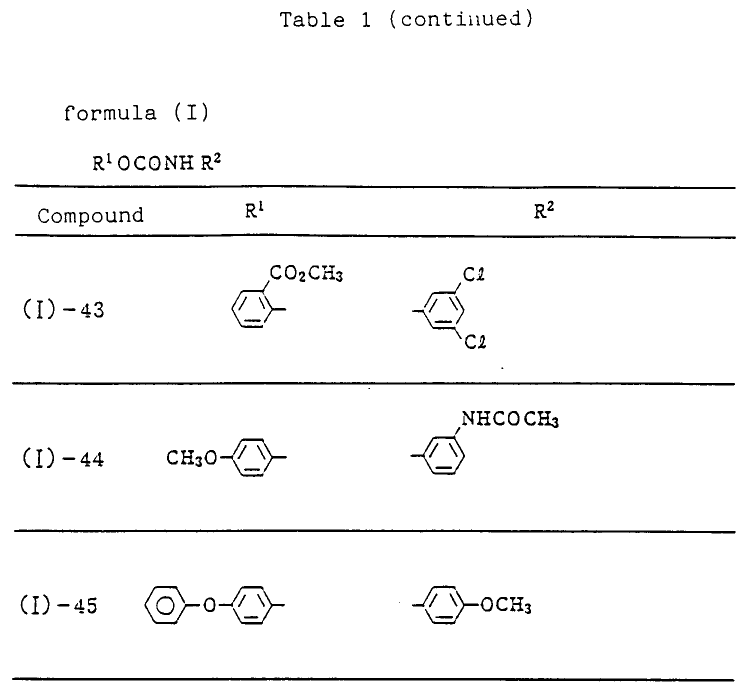

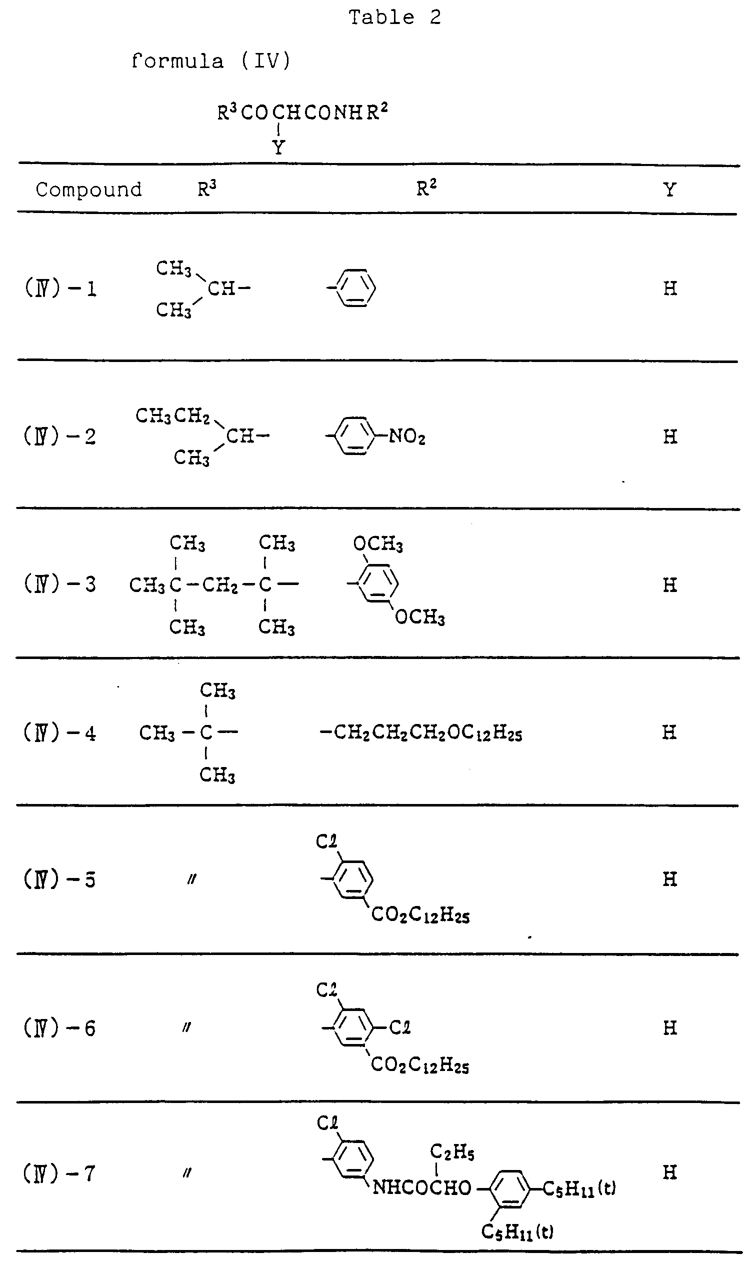

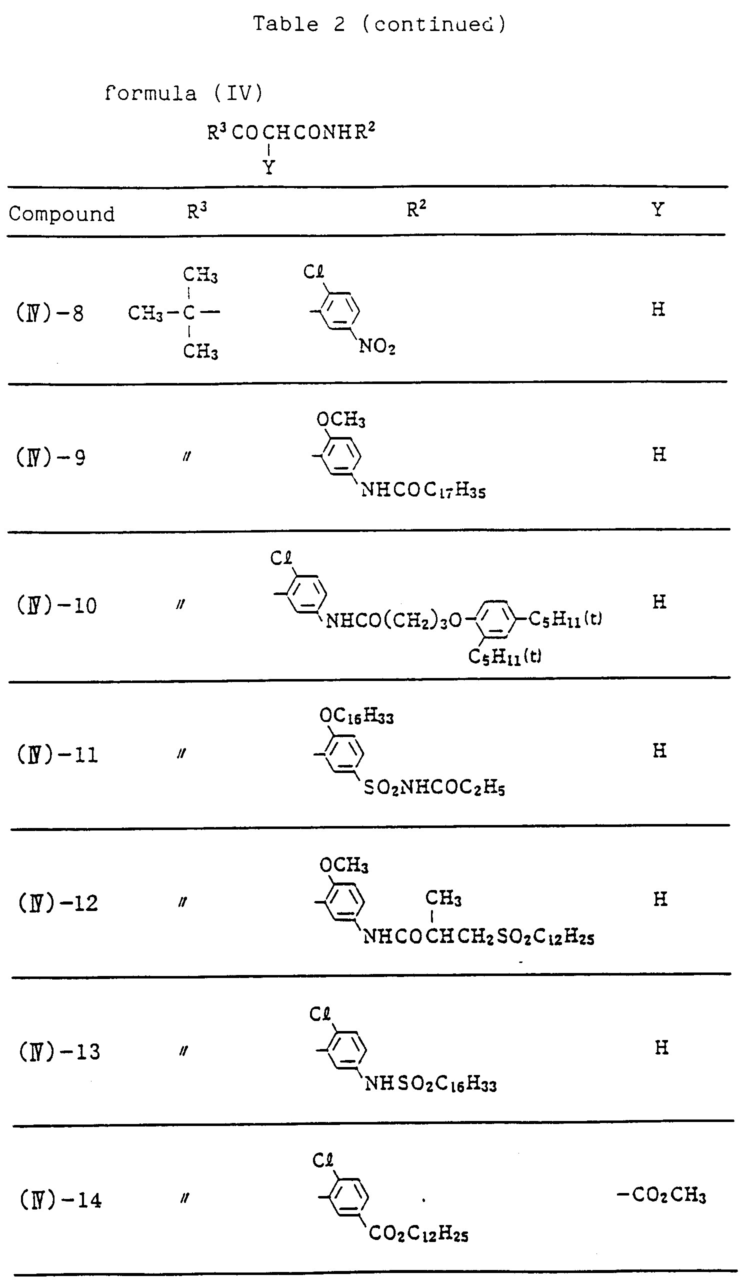

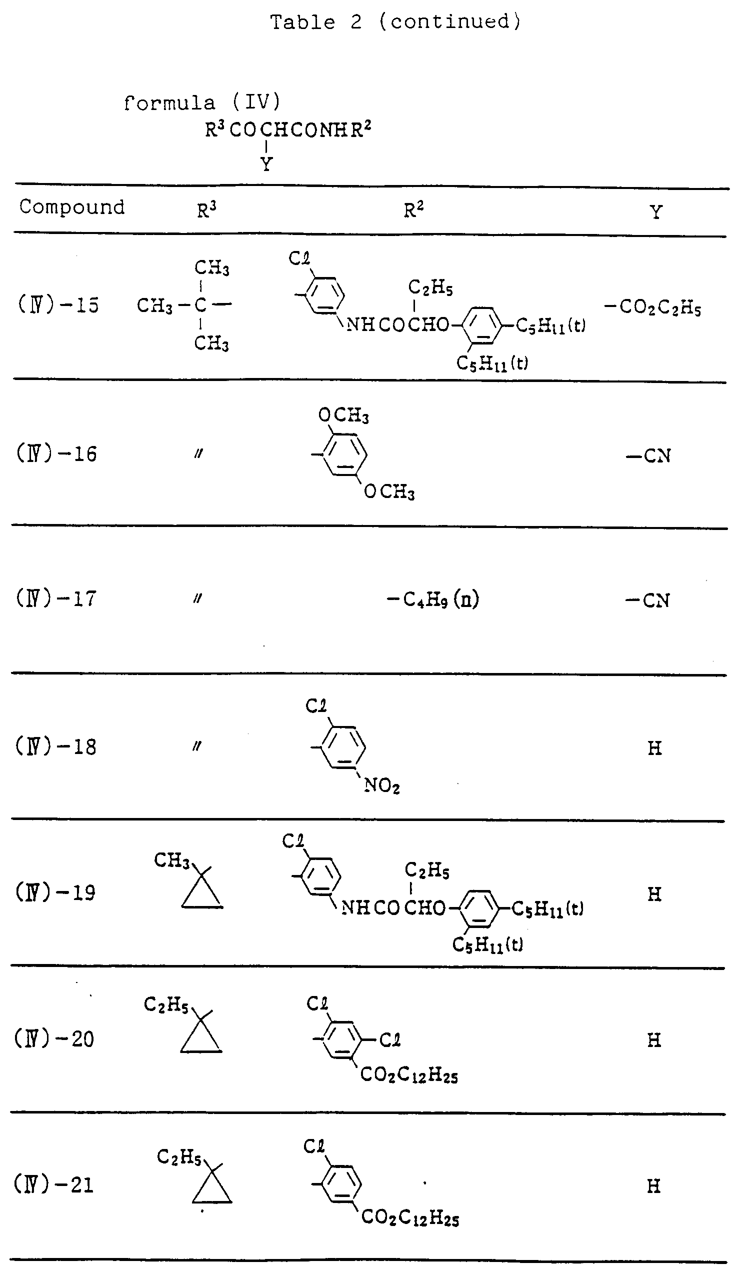

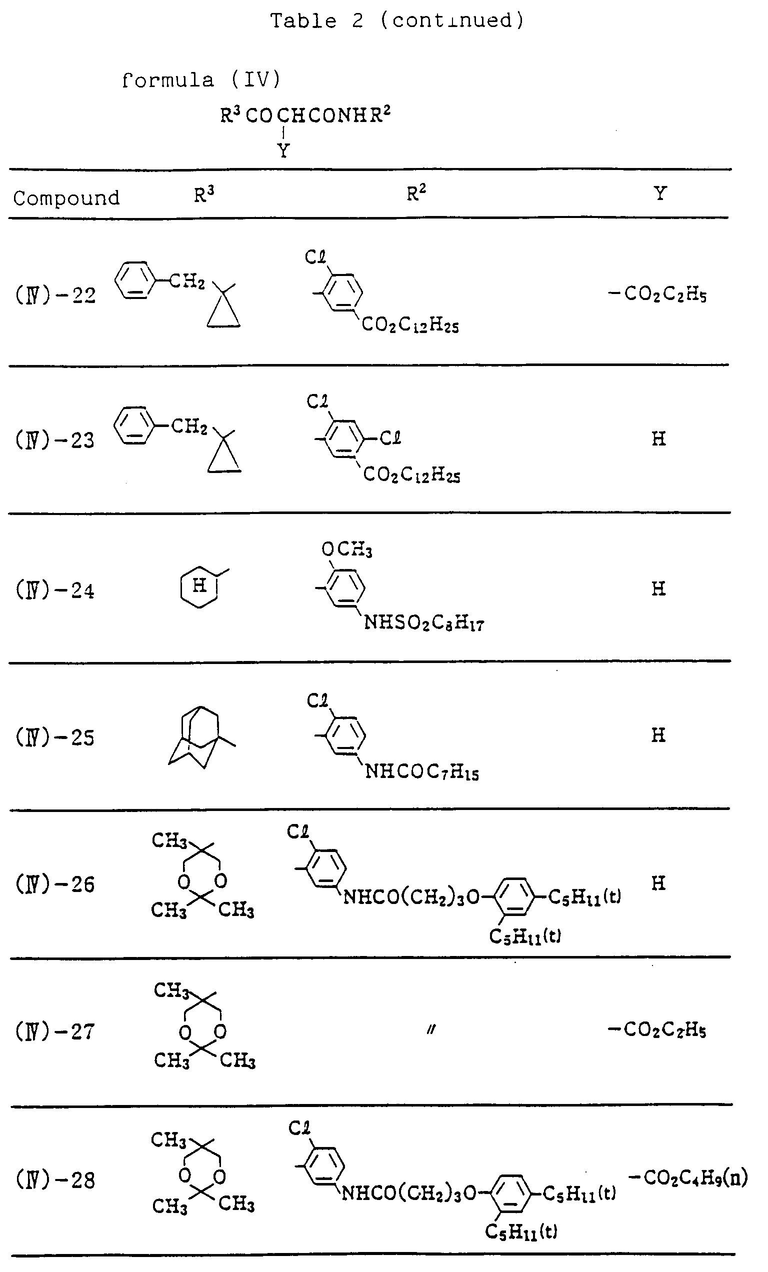

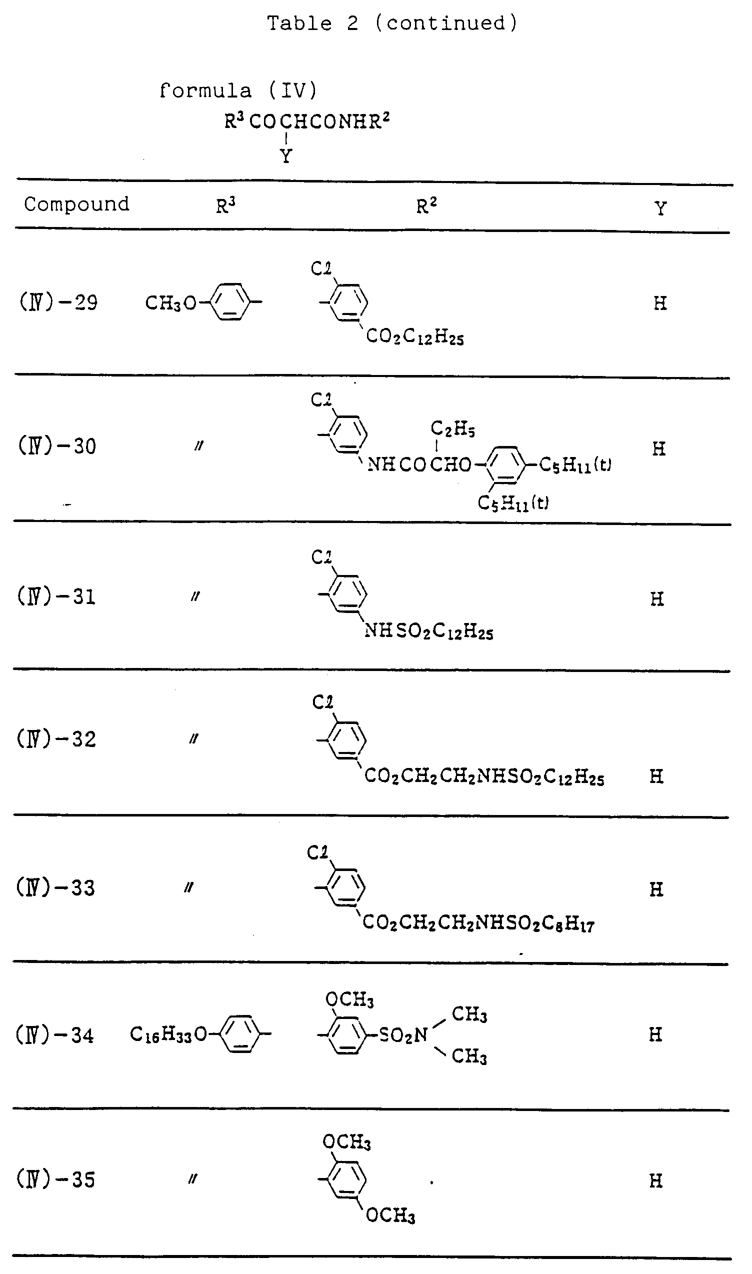

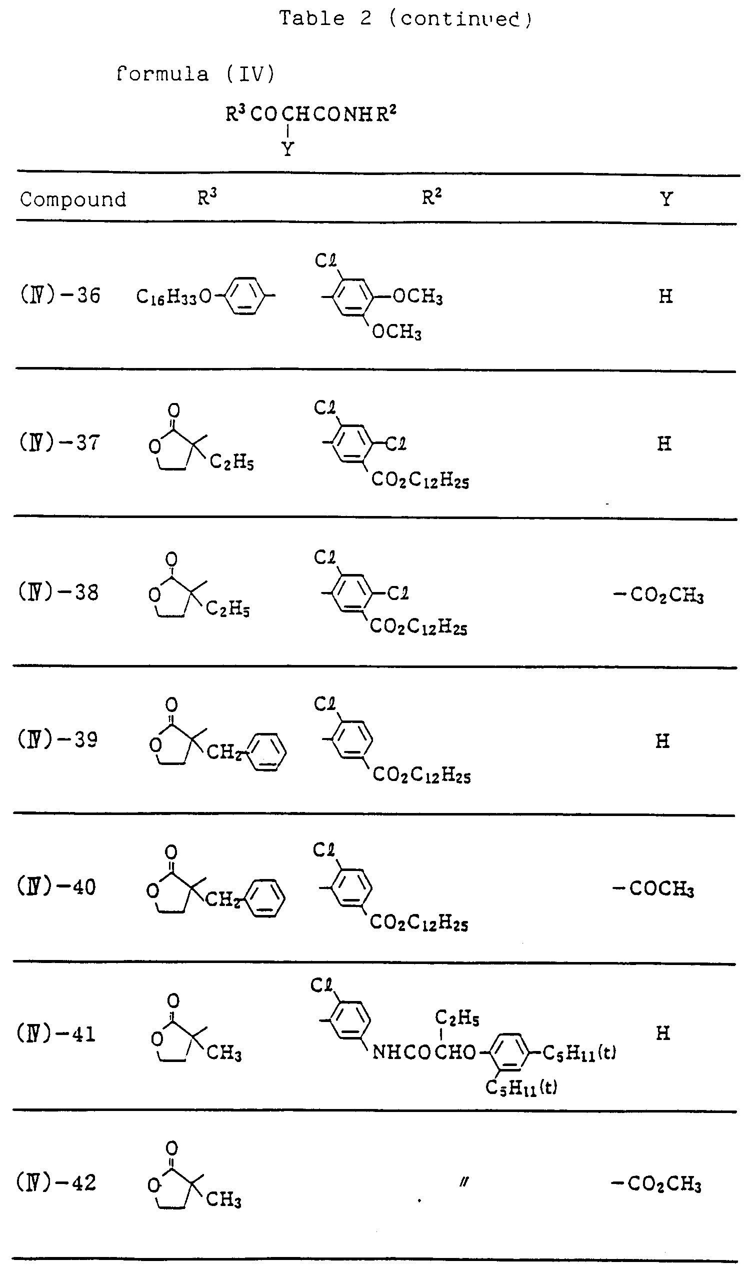

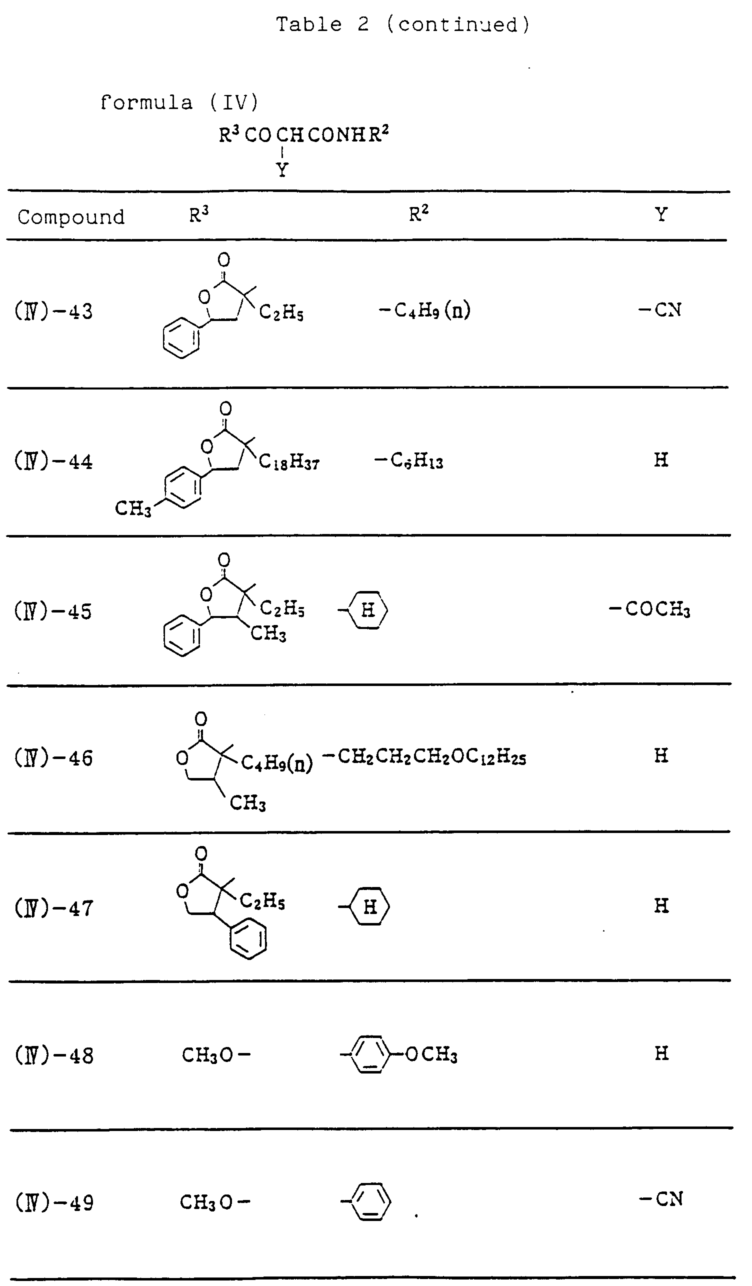

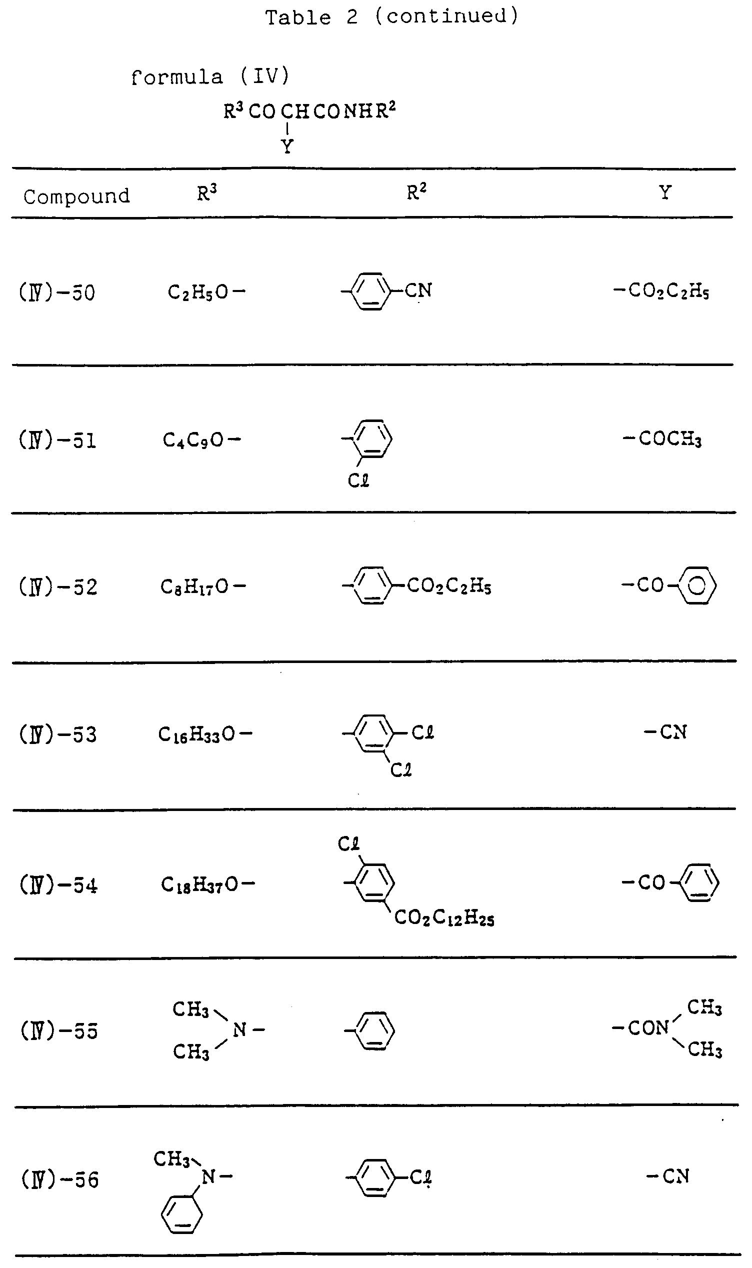

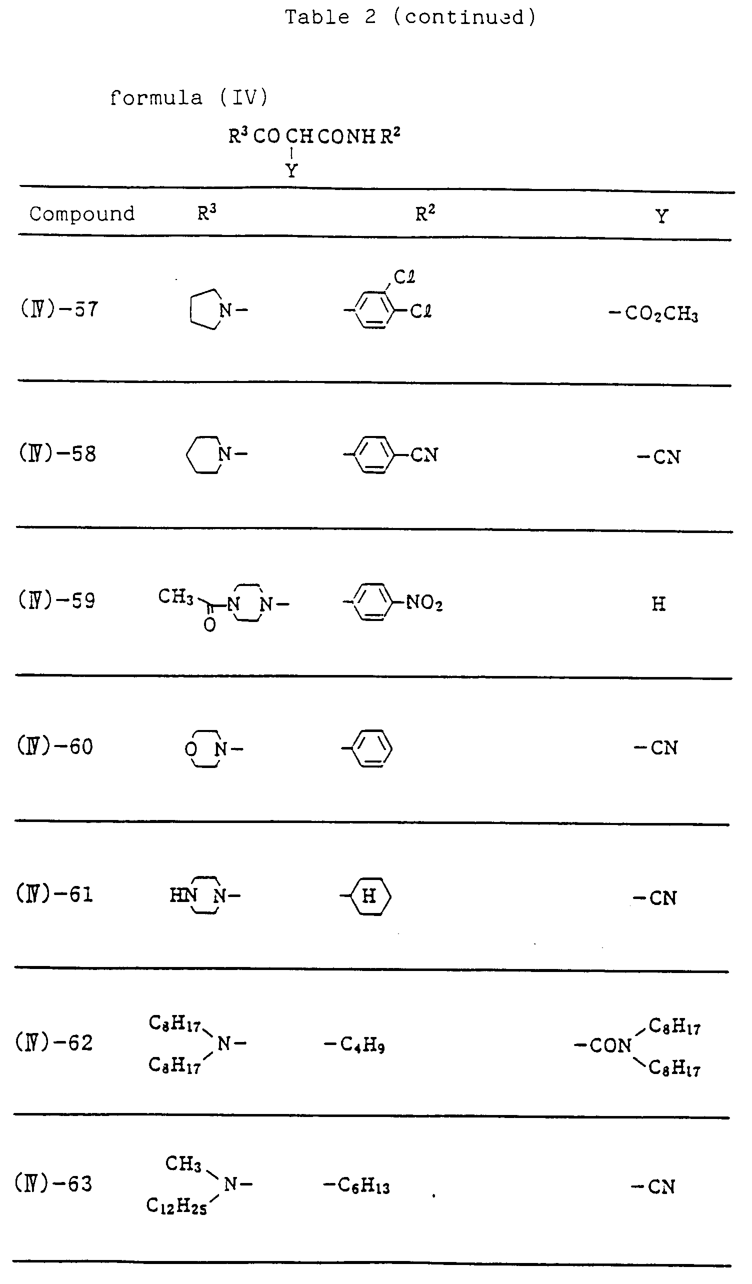

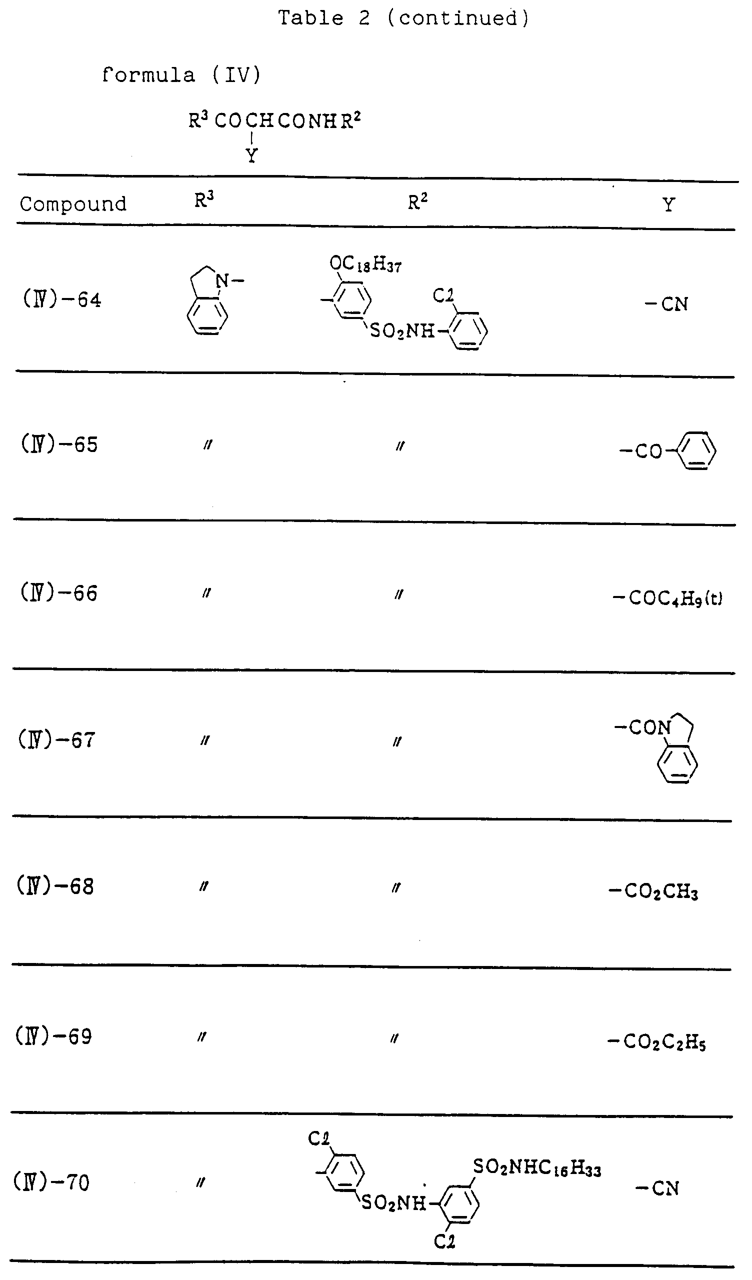

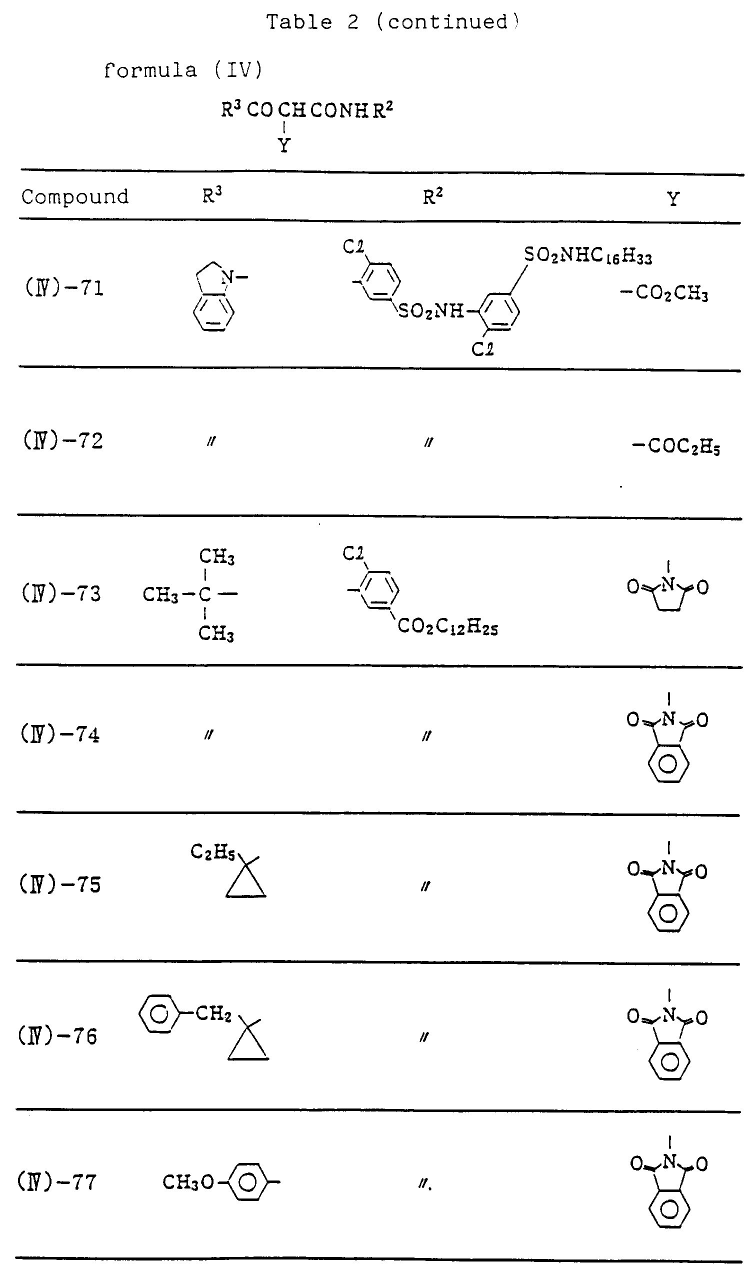

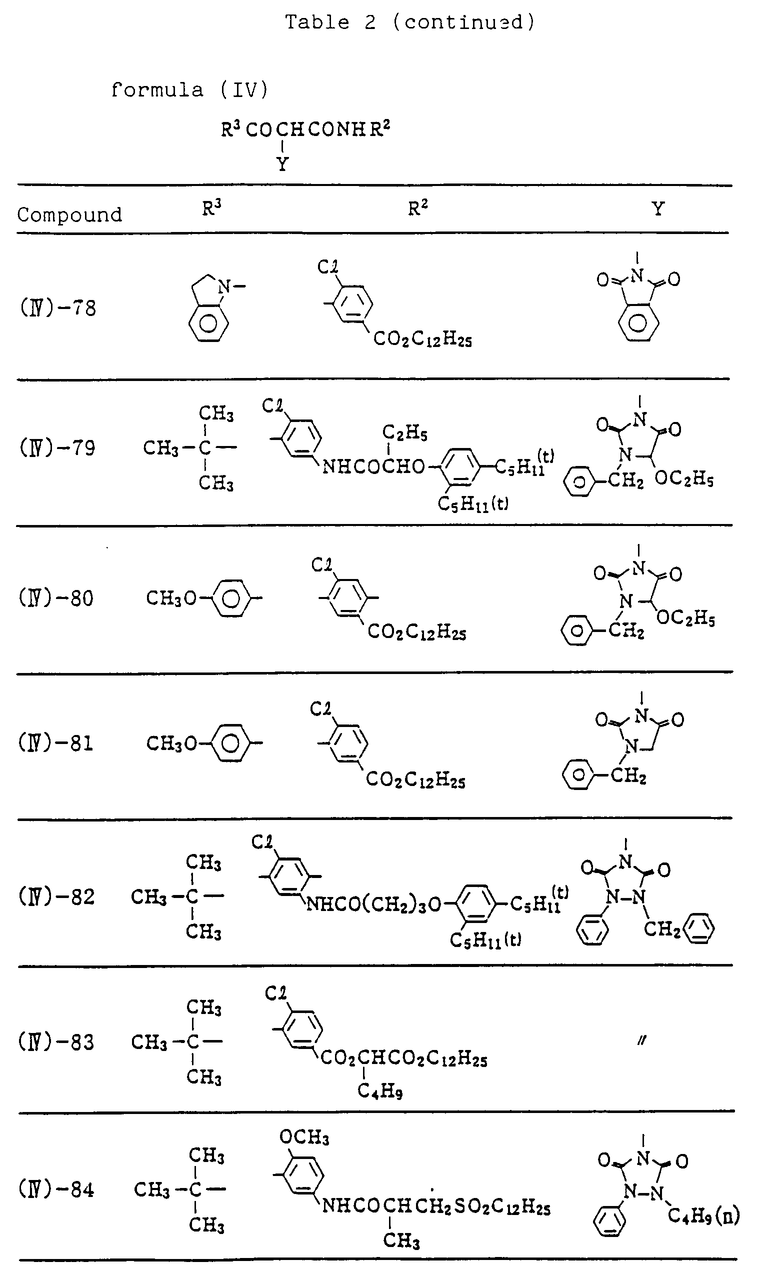

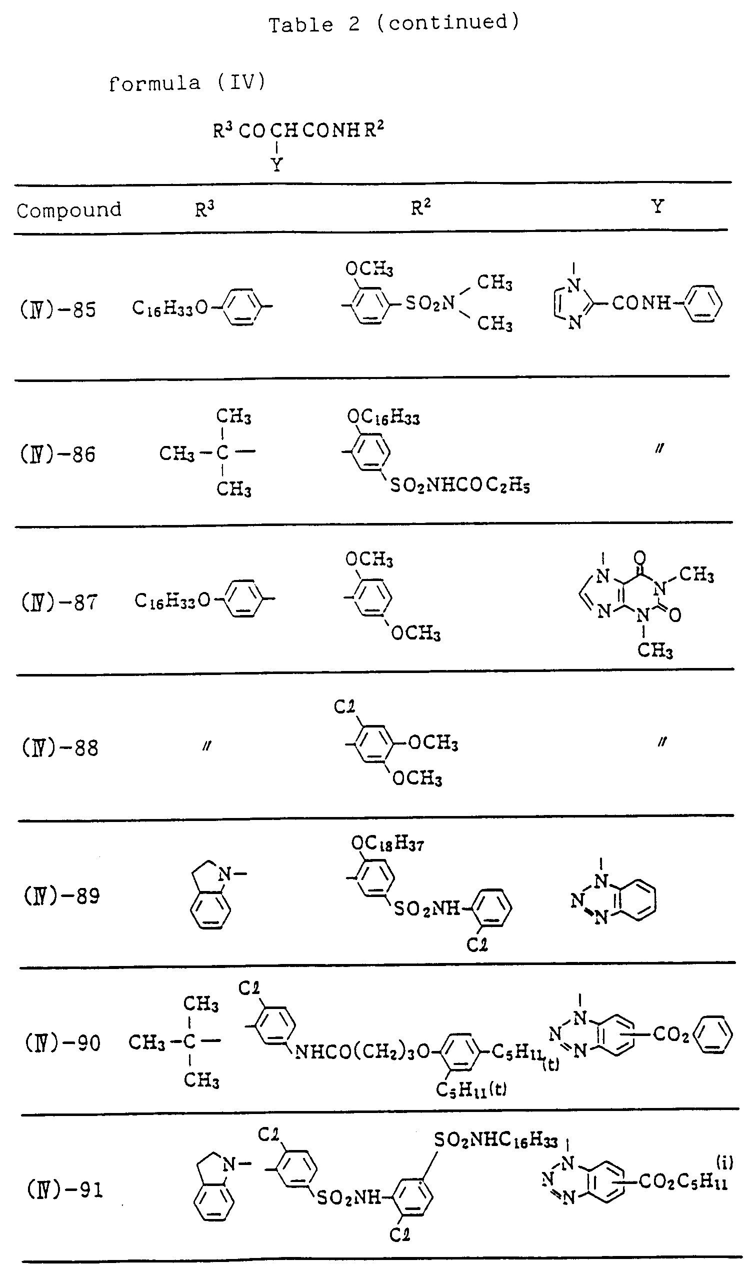

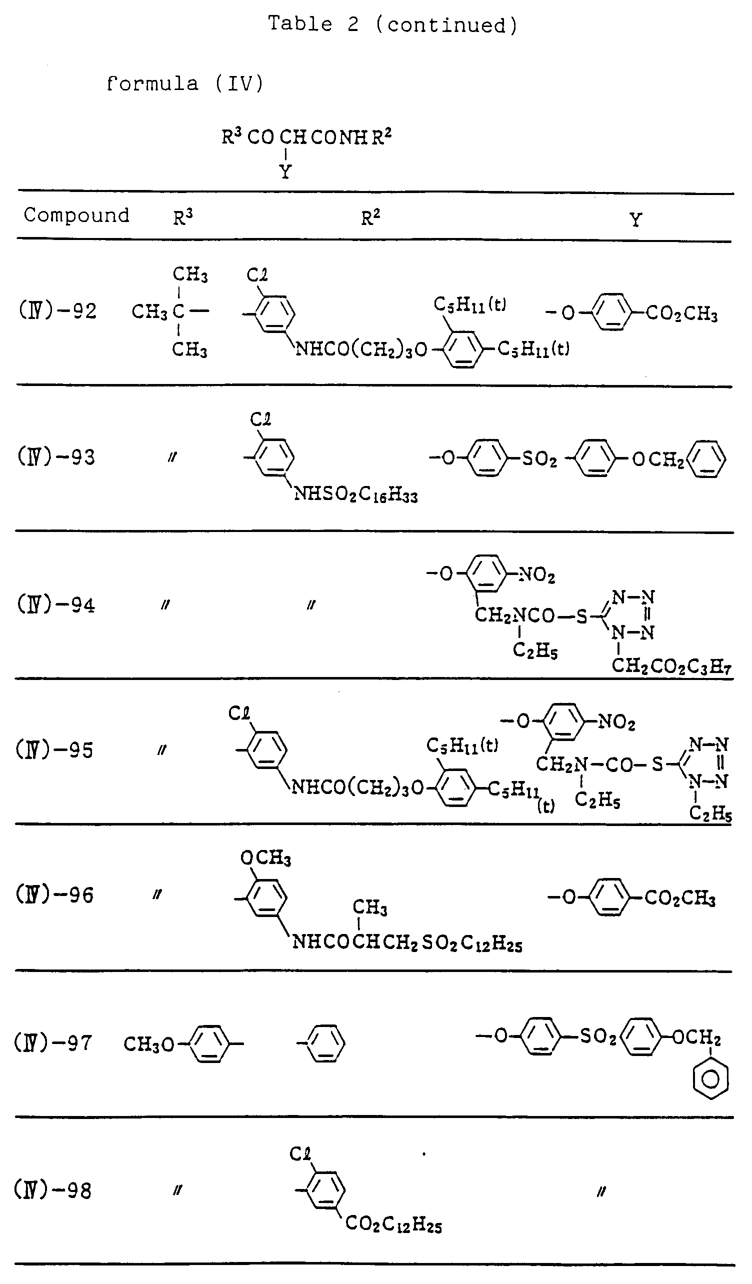

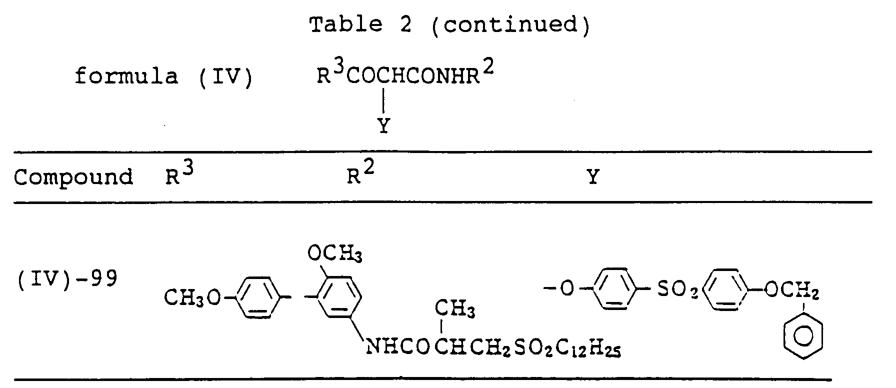

The compound of the second invention of the present invention will now be described in detail below.

R¹⁶ represents a tert-butyl group or a phenyl group having an alkoxy group with 1 to 18 carbon atoms (e.g., methoxy, ethoxy, hexadecyloxy, and octadecyloxy) in the paraposition. More preferably R¹⁶ represents a tert-butyl group, a p-methoxyphenyl group, a p-hexadecyloxyphenyl group, or a p-octadecyloxyphenyl group, with particular preference given to a tert-butyl group and a p-hexadecyloxyphenyl group.

R¹⁸ represents an alkoxy group having 1 to 18 carbon atoms, preferably 1 to 6 carbon atoms (e.g., methoxy, hexyloxy, and hexadecyloxy), or an anilino group.

When R¹⁸ represents an alkoxy group, the substitution position of the group -COR¹⁸ is preferably the 4-position or 5-position of the said imidazole ring. At that time, R¹⁶ is particularly preferably a tert-butyl group.

When R¹⁸ represents an anilino group, the substitution position of the group -COR¹⁸ is preferably the 2-position of the said imidazole ring.

R¹⁹ and R²⁰ each represent an alkyl group having 1 to 18 carbon atoms (e.g., methyl, ethyl, propyl, butyl, and octadecyl).

Preferably R¹⁹ and R²⁰ each represent a lower alkyl group, more preferably a methyl group.

Particularly preferable combinations of substituents in the compound of the second invention are shown below.
(1) R¹⁶ represents a tert-butyl group or a phenyl group having an alkoxy group with 1 to 18 carbon atoms in the paraposition; R¹⁷ represents a 1-imidazolyl group having a group -COR¹⁸ in the 2-position; and R¹⁸ represents an anilino group.
(2) R¹⁶ represents a phenyl group having an alkoxy group with 1 to 18 carbon atoms in the paraposition; R¹⁷ represents a group of formula (XI); and R¹⁹ and R²⁰ each represent a lower alkyl group.
(3) R¹⁹ and R²⁰ in the above (2) each represent a methyl group.
(4) R¹⁶ represents a tert-butyl group or a phenyl group having an alkoxy group with 1 to 18 carbon atoms in the paraposition; R¹⁷ represents a group of formula (X); -COR¹⁸ is in the 4-position or 5-position of the imidazole ring; and R¹⁸ represents an alkoxy group having 1 to 18 carbon atoms.
(5) R¹⁶ in the above (4) represents a tert-butyl group.

Now the production method of the compound of the second invention will be described.

The compound of the second invention can be obtained in a good yield, as shown below, by reacting an α-haloacetyl compound with a nitrogen-containing heterocyclic ring under basic conditions. The reaction molar ratio of the α-haloacetyl compound to the nitrogen-containing heterocyclic ring is generally from 1 : 5 to 5 : 1, preferably from 1 : 2 to 2 : 1. 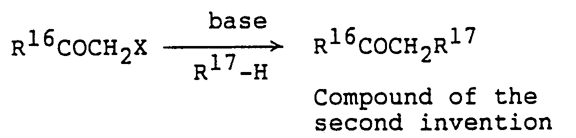 wherein R¹⁶, R¹⁷, and X have the same meanings as defined above.

As the base to be used in the reaction, an inorganic base, such as sodium hydride, potassium tert-butoxide, sodium carbonate, potassium carbonate, sodium methoxide, sodium ethoxide, sodium hydroxide, and potassium hydroxide, and an organic base, such as 1,8-diazabicyclo[5.4.0]-7-undecene (DBU), 1,5-diazabicyclo[4.3.0]-5-nonene (DBN), N,N-diisopropylethylamine, and triethylamine, can be mentioned. The amount of the base to be used is not particularly restricted, but generally it is 0.5 to 2 equivalents, preferably 0.8 to 1.2 equivalents, based on the quantity of the nitrogen-containing heterocyclic ring. As the reaction solvent, for example, ether, tetrahydrofuran (THF), dioxane, acetonitrile, N,N-dimethylformamide (DMF), N,N-dimethylacetamide (DMAc), 1,3-dimethyl-2-imidazolidone (DMI), and 1-methyl-2-pyrrolidone (NMP) can be used. The reaction temperature is 0 to 140°C, preferably 10 to 100°C. There is no restriction on the reaction time, but generally it is 0.5 to 10 hours, preferably 1 to 5 hours.

The compound of the second invention may be useful as a synthetic intermediate of a photographic yellow coupler, as shown below. 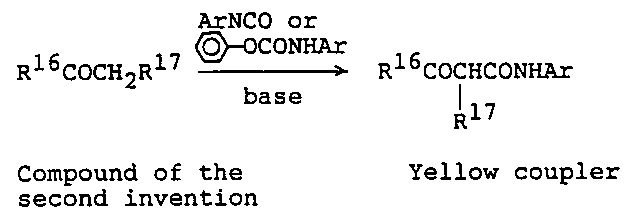 wherein R¹⁶ and R¹⁷ have the same meanings as defined above, and Ar represents an aromatic group.

One mol of the compound of the second invention is reacted with 0.5 to 2 mol, preferably 0.8 to 1.2 mol, of an isocyanate compound or its precursor, a phenylurethane compound, in the presence of a base, to produce a yellow coupler in a good yield.

The isocyanate compound is easily synthesized from the corresponding aniline derivative and phosgene. The phenylurethane compound is easily synthesized from the corresponding aniline derivative and phenyl chlorocarbonate.

As the base to be used, for example, LDA (lithium diisopropylamide), sodium hydride, potassium tert-butoxide, and sodium methoxide can be mentioned.

The amount of the base to be used is 0.9 to 5 mol, preferably 1 to 3 mol, based on the compound of the second invention.

The method for synthesizing a β-ketoanilide compound from an acetyl compound and an isocyanate compound is known, and typical known processes are those shown as (A) and (B) above.

The compound of the second invention is an intermediate in the production method of the first invention.

Specific examples of the compound of the second invention are shown below, but the compound of the present invention is not limited to them.

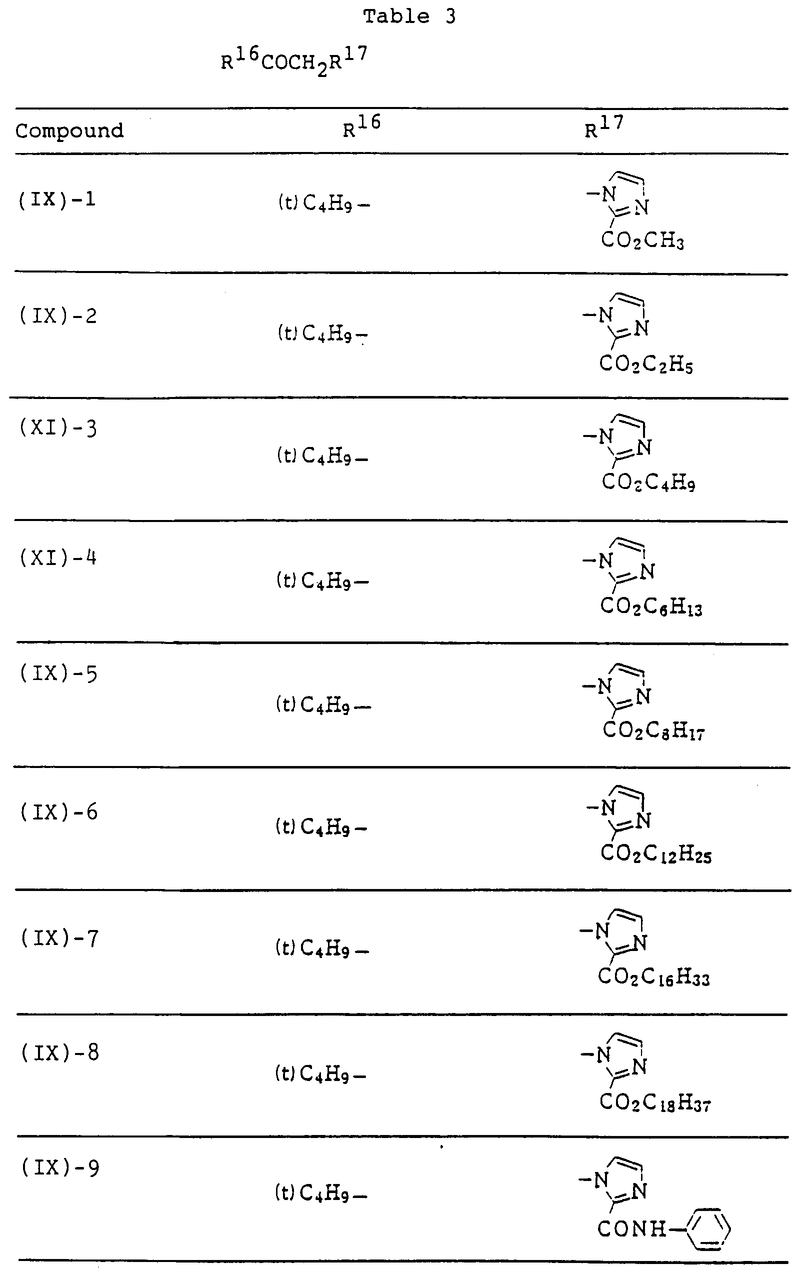

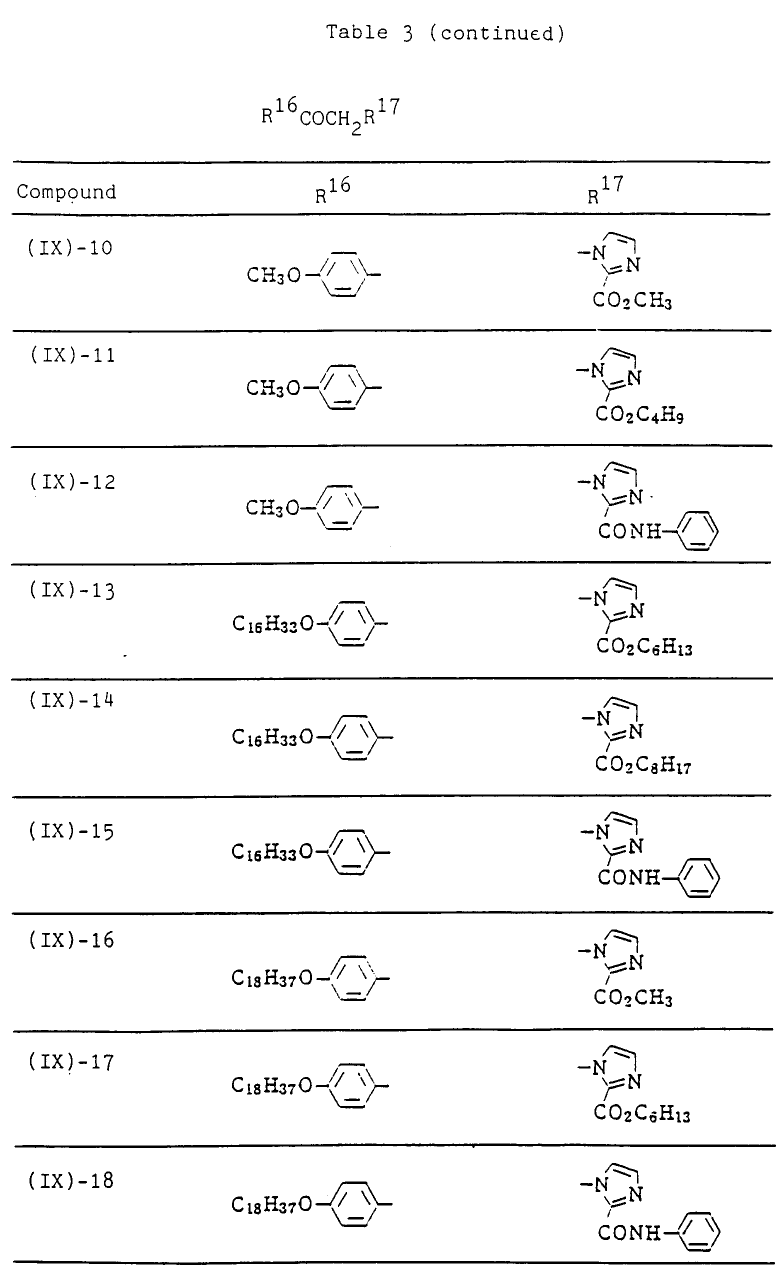

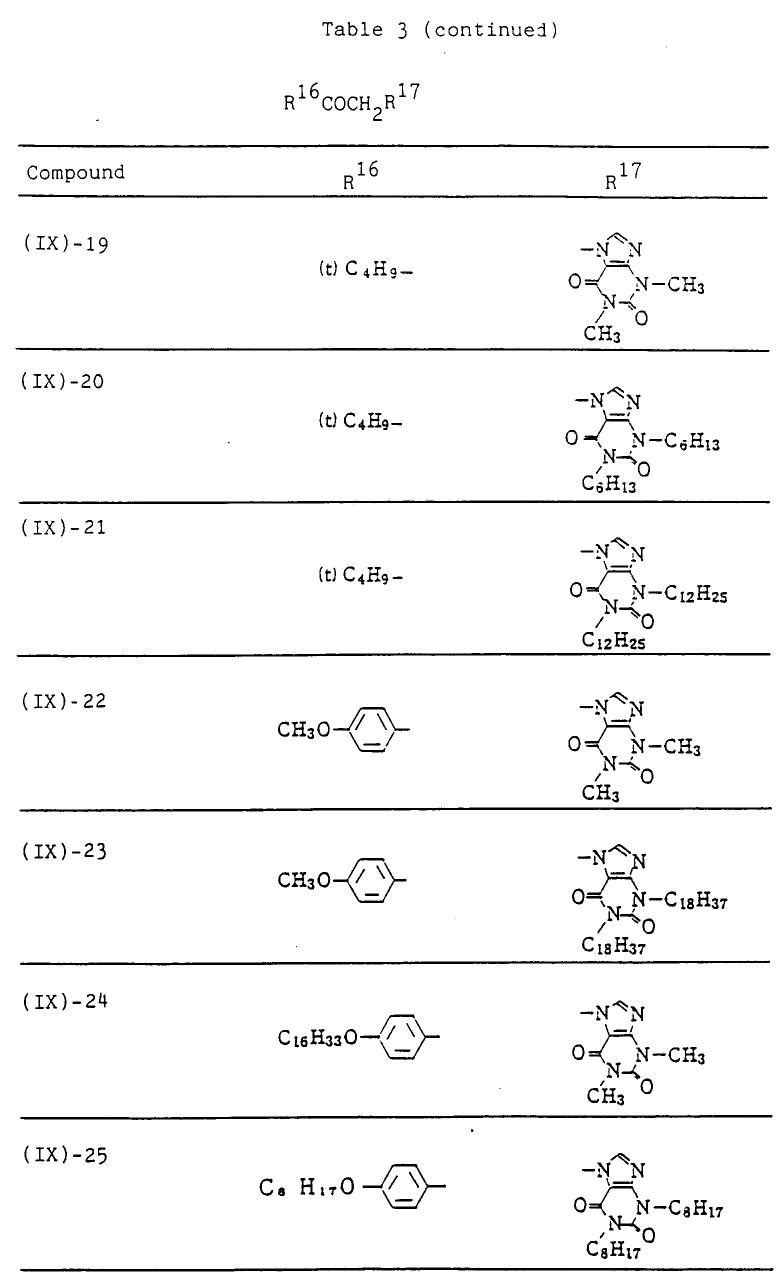

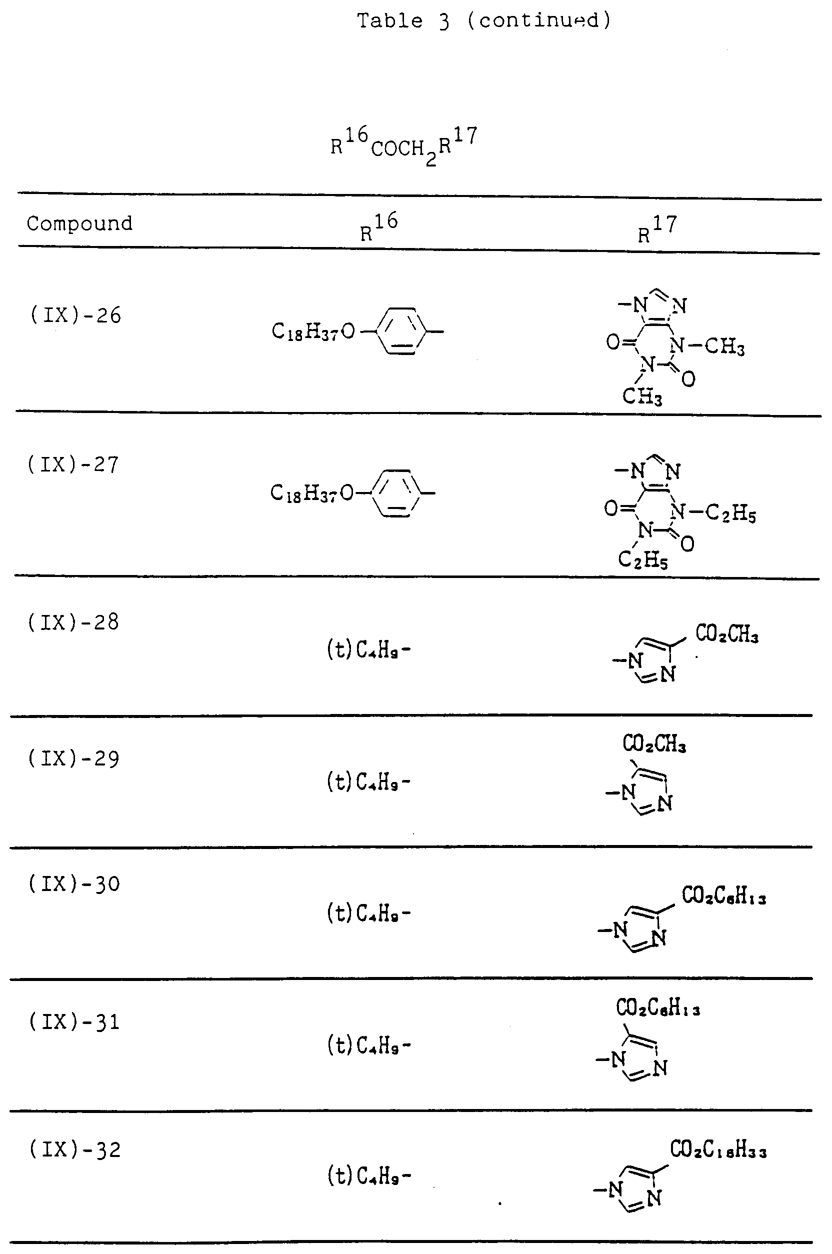

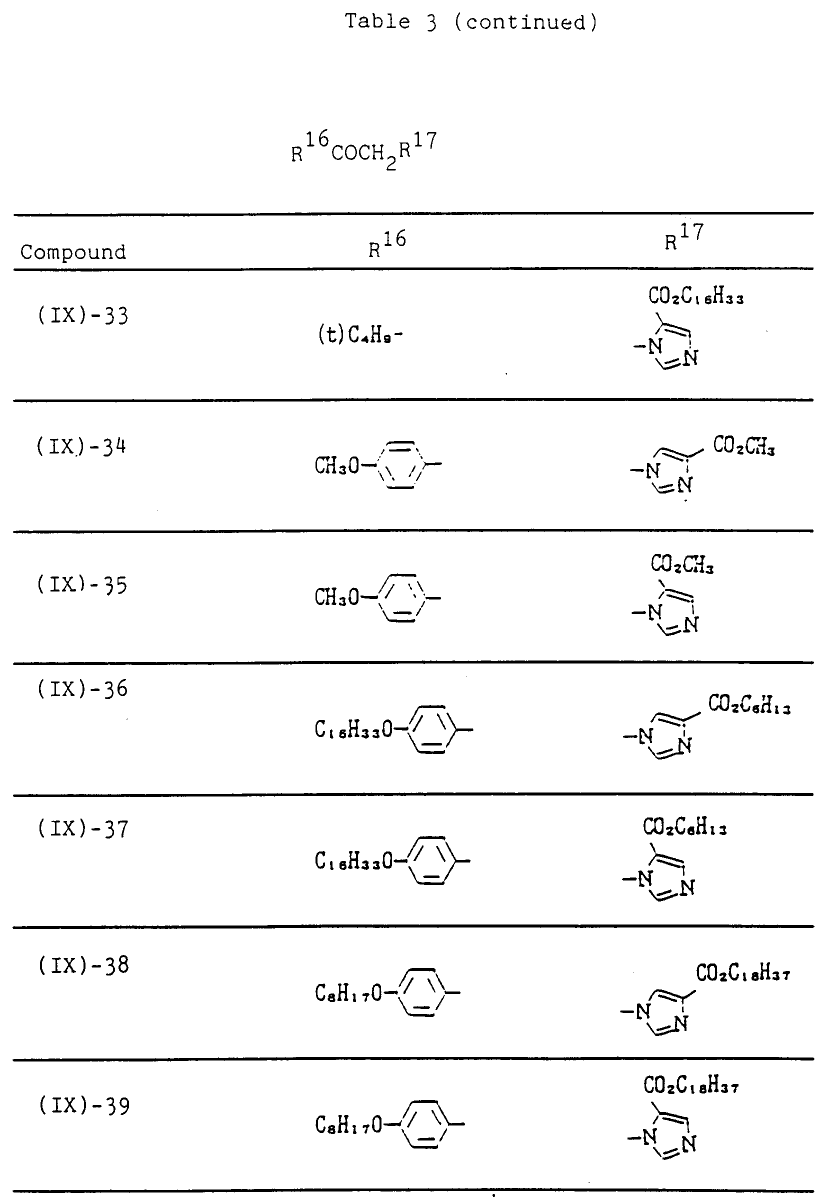

According to the first invention of the present invention, a compound useful as a yellow coupler of silver halide color photographic materials and a synthetic intermediate of the coupler can be simply synthesized under mild conditions without using a highly toxic compound.

The compound of the second invention of the present invention may be used as a synthetic intermediate from which a yellow coupler can be synthesized by a simple method. By using the compound of the second invention, a yellow coupler can be produced easily on an industrial scale with the process shortened.

The present invention will now be described in more detail based on the following Examples, but the present invention is not restricted to them.

### Reference Example 1 (Synthesis of (I)-11)

170.0 g (0.5 mol) of 2-chloro-5-dodecyloxycarbonylaniline was dissolved in 500 ml of N,N-dimethylformamide; then 50.0 ml (0.064 mol) of pyridine was added to the solution, and 68.7 ml (0.6 mol) of phenyl chlorocarbonate was added thereto, with the internal temperature being 12 to 18°C under cooling with ice, followed by allowing the reaction mixture to stand overnight. One liter of water and 500 ml of ethyl acetate were added thereto, and the organic layer was separated, washed with water twice, and dried over magnesium sulfate. The solvent was distilled off, to obtain a yellow oil, which solidified gradually. Two liters of acetonitrile was added to the solid, followed by heating, to dissolve the solid completely; the solution was cooled with water, and the deposited crystals were filtered, to obtain colorless crystals of (I)-11, in an amount of 196.0 g (0.425 mol). Yield: 85%. Melting point: 57 to 58°C.

Other compounds of formula (I) can also be obtained from the corresponding aniline and aryl chlorocarbonate in good yields. The melting points of the typical examples are shown below:

| | |
|---|---|
| (I)-10 | 125-126°C |
| (I)-12 | 70-71°C |
| (I)-16 | 83-85°C |
| (I)-19 | 81-83°C |
| (I)-24 | 92-94°C |

### Example 1 (Synthesis of (IV)-5 by Using (I)-11)

8.8 g (0.22 mol) of sodium hydride (60%) was dispersed in 100 ml of tetrahydrofuran, and while the dispersion was heated in a nitrogen atmosphere under reflux, a solution of 12.0 g (0.12 mol) of pinacolone and 46.0 g (0.1 mol) of (I)-11 in 250 ml of tetrahydrofuran was added, dropwise, to the dispersion over 20 min, and thereafter the reaction mixture was heated under reflux for 2 hours. The temperature was brought to room temperature, 1 liter of water was added, dropwise, to the reaction liquid, and the reaction liquid was acidified with 20 ml of concentrated hydrochloric acid. Extraction with ethyl acetate was carried out; the extract was washed with water; and the solvent was removed by distillation. Three hundred ml of ethanol was added to the residue, followed by cooling with ice; the deposited crystals were filtered off, and the filtrate was condensed, purified by silica gel column chromatography (n-hexane/ethyl acetate = 5/1), and crystallized from n-hexane, to obtain 17.5 g of colorless crystals of (IV)-5. Yield: 38%. Melting point: 49 to 51°C.

### Example 2 (Synthesis of (IV)-29 by Using (I)-11)

Example 1 was repeated, except that, in place of 12.0 g of pinacolone, 16.5 g (0.11 mol) of p-methoxyacetophenone was used, thereby obtaining 26.2 g of colorless crystals of (IV)-29. Yield: 50%. Melting point: 140 to 142°C.

### Example 3 (Synthesis of (IV)-37 by Using (I)-12)

5.2 g of sodium hydride (60%) was dispersed in 150 ml of tetrahydrofuran; then 11.7 g (0.075 mol) of 2-acetyl-2-ethyl-γ-butyrolactone, easily obtainable by ethylation of 2-acetyl-γ-butylolactone, was added to the dispersion in a nitrogen atmosphere under cooling with ice, and 100 ml of a solution containing 24.7 g (0.05 mol) of (I)-12 in tetrahydrofuran was added, dropwise, over 30 min, with the internal temperature kept at 15°C or below. After the addition, the reaction was continued for 4 hours; then the reaction mixture was poured into 1.5 liters of ice water, to which 11.0 ml of concentrated hydrochloric acid had been added, followed by extraction with ethyl acetate, and the extract was washed with water and dried over magnesium sulfate. After the solvent was distilled off, the residue was purified by silica gel column chromatography (n-hexane/ethyl acetate = 3/1), thereby obtaining 22.5 g of a pale yellow oil of (IV)-37. Yield: 81%.
- mass: 559 (M⁺ +4)
557 (M⁺ +2)
555 (M⁺)
- ¹H-NMR: (CDCl₃: 200 MHz)
- δppm: 0.90 (t, 3H, J = 7.0 Hz)
1.05 (t, 3H, J = 7.7 Hz)
1.23 (m, 18H)
1.40 (m, 2H)
1.77 (m, 2H)
2.17 (m, 1H)
3.00 (m, 1H)
3.90 (d, 1H, J = 16.7 Hz)
4.00 (d, 1H, J = 16.7 Hz)
4.30 (m, 4H)
7.50 (s, 1H)
8.84 (s, 1H)
9.18 (s, 1H)

### Example 4 (Synthesis of (IV)-64 by Using (I)-16)

Cyanoacetylindoline was synthesized as shown below:

11.9 g (0.1 mol) of indoline and 13.4 g (0.11 mol) of ethyl cyanoacetate were mixed and stirred for 2 hours on an oil bath at a temperature of 140 to 150°C; then they were stirred for a further 5 hours under slightly reduced pressure created by an aspirator, to remove the produced ethanol.

Then, the heating was stopped; 50 ml of ethanol was added to the reaction mixture; the temperature was brought to room temperature, and the deposited crystals were filtered, to obtain 10.8 g of cyanoacetylindoline. Yield: 58%.

0.40 g of sodium hydride (60%) was dispersed in 5 ml of N,N-dimethylformamide; then 0.56 g (3 mmol) of the cyanoacetylindoline was added to the dispersion under a nitrogen atmosphere; then 10 ml of a solution of 2.01 g (3 mmol) of (I)-16 in N,N-dimethylformamide was added under cooling with ice, and they were reacted for 2 hours. The reaction mixture was poured into acidic water, followed by extraction with ethyl acetate; the extract was washed with water; the solvent was removed by distillation, and the residue was crystallized from n-hexane/ethyl acetate, to obtain 2.28 g of (IV)-64. Yield: 99%. Melting point: 149 to 151°C.

### Example 5 (Synthesis of (IV)-68 by Using (I)-16)

Methoxycarbonylacetylindoline was synthesized by the method shown below.

460 g (3.5 mol) of dimethyl malonate was heated, to make the internal temperature 130°C, and 60.0 g (0.5 mol) of indoline was added, dropwise, over 2 hours. After the addition, the mixture was stirred for 2 hours; the excess dimethyl malonate was distilled off under reduced pressure; then 300 ml of acetonitrile was added to the residue, followed by stirring under cooling with ice. The deposited crystals were filtered off, and the filtrate was condensed and then purified by column chromatography (n-hexane/ethyl acetate = 1/1), to obtain 80.0 g of methoxycarbonylacetylindoline. Yield: 73%.

0.40 g of sodium hydride (60%) was dispersed in 5 ml of tetrahydrofuran, and then a solution of 0.66 g (3 mmol) of the methoxycarbonylacetylindoline and 2.01 g (3 mmol) of (I)-16 in 10 ml of tetrahydrofuran was added, dropwise, to the dispersion over 20 min in a nitrogen atmosphere under cooling with ice, with the internal temperature kept at 5 to 10°C, followed by stirring for 1 hour. The reaction mixture was poured into ice water and neutralized, followed by extraction with ethyl acetate; then the extract was washed with water; the solvent was removed by distillation, and the residue was purified by column chromatography (n-hexane/ethyl acetate = from 4/1 to 1/1), to obtain 2.22 g of amorphous (IV)-68. Yield: 93%.
- ¹H-NMR: (CDCl₃: 300 MHz)
- δppm: 0.86 (t, 3H, J = 7.5 Hz)
1.17-1.44 (m, 30H)
1.50-1.62 (m, 2H)
1.84-1.97 (m, 2H)
3.26 (t, 2H, J = 9.0 Hz)
3.80 (s, 3H)
4.00-4.12 (m, 2H)
4.25-4.45 (m, 2H)
4.75 (s, 1H)
6.82 (d, 1H, J = 9.0 Hz)
6.97 (t, 1H, J = 9.0 Hz)
7.05 (s, 1H)
7.10 (t, 1H, J = 9.0 Hz)
7.15-7.27 (m, 4H)
7.50 (d, 1H, J = 9.0 Hz)
7.63 (d, 1H, J = 9.0 Hz)
8.21 (d, 1H, J = 9.0 Hz)
8.88 (s, 1H)
10.50 (s, 1H)

### Example 6 (Synthesis of (IV)-79 by Using (I)-13)

56.2 g (0.24 mol) of 1-benzyl-5-ethoxyimidazolidin-2,4-dione and 26.8 g (0.2 mol) of α-chloropinacolone were dissolved in 150 ml of acetonitrile, and then 33.6 ml (0.24 mol) of triethylamine was added to the solution, followed by heating for 5 hours under reflux. The temperature was brought to room temperature, 500 ml of ethyl acetate and 800 ml of water were added thereto, and the organic layer was separated and washed with saturated brine. After the solvent was distilled off, the residue was purified by column chromatography (n-hexane/ethyl acetate = 4/1), to obtain 52.5 g of a colorless oil of 1-benzyl-5-ethoxy-3-pivaloylmethylimidazolidin-2,4-dione. Yield: 79%.

Then, 0.46 g (11.5 mmol) of sodium hydride (60%) was added to 5 ml of tetrahydrofuran; then 1.50 g (4.5 mmol) of the above-obtained 1-benzyl-5-ethoxy-3-pivaloylmethylimidazolidin-2,4-dione was added thereto, at an internal temperature of 5 to 10°C under cooling with ice, and a solution of 1.70 g (3 mmol) of (I)-13 in 10 ml of tetrahydrofuran was added thereto, dropwise, over 20 min. After completion of the addition, the reaction mixture was stirred for 3 hours; then ice-hydrochloric acid was added, followed by extraction with ethyl acetate, and the extract was washed with water. After the solvent was distilled off, the residue was purified by column chromatography (n-hexane/ethyl acetate = 4/1) and crystallized from n-hexane, to obtain 0.96 g of colorless crystals of (IV)-79. Yield: 40%. Melting point: 98 to 104°C.

### Example 7 (Synthesis of (IV)-80 by Using (I)-11)

5.11 g (21.8 mmol) of 1-benzyl-5-ethoxyimidazolidin-2,4-dione was dissolved in 15.0 ml of N,N-dimethylformamide; then 3.0 ml of triethylamine was added to the solution, and then a solution of 5.00 g (21.8 mmol) of 4'-methoxyphenacyl bromide in 15.0 ml of N,N-dimethylformamide was added thereto, dropwise, over 30 min. After completion of the addition, the reaction mixture was stirred for 7 hours at room temperature; then ethyl acetate and water were added thereto, the organic layer was separated and washed with water, and the solvent was removed by distillation. The thus obtained oil was purified by column chromatography (n-hexane/ethyl acetate = 3/2), to obtain 6.51 g of a colorless oil of 1-benzyl-5-ethoxy-3-(4'-methoxybenzoylmethyl)imidazolidin-2,4-dione. Yield: 79%.

Then, 0.32 g (8 mmol) of sodium hydride (60%) was added to 5.0 ml of tetrahydrofuran; then a solution of 1.72 g (4.50 mmol) of the 1-benzyl-5-ethoxy-3-(4'-methoxybenzoylmethyl)imidazolidin-2,4-dione in 7.0 ml of tetrahydrofuran was added to the resulting mixture, over 20 min under a nitrogen atmosphere under cooling with ice with the internal temperature being 5 to 10°C, and then a solution of 1.38 g (3 mmol) of (I)-11 in 7.0 ml of tetrahydrofuran was added thereto over 30 min. After completion of the addition, the reaction mixture was stirred for 3 hours; then ice-hydrochloric acid was added thereto, followed by extraction with ethyl acetate; the extract was washed with water; the solvent was removed by distillation, to obtain an oil, which was purified by column chromatography (n-hexane/ethyl acetate = 3/1) and was crystallized from n-hexane/ethyl acetate, to obtain 1.37 g of colorless crystals of (IV)-80. Yield: 61%. Melting point: 87 to 90°C.

### Example 8 (Synthesis of (IV)-87 by Using (I)-24)

27.2 g (0.2 mol) of 4'-hydroxyacetophenone and 61.0 g (0.2 mol) of hexadecyl bromide were dissolved in N,N-dimethylformamide; then 27.8 g (0.2 mol) of potassium carbonate was added to the solution, and the resulting mixture was heated on a steam bath for 4 hours with stirring. The temperature was brought to room temperature; then 500 ml of water was added thereto under cooling with water; the deposited crystals were filtered, to obtain 70.0 g of 4'-hexadecyloxyacetophenone. Yield: 97%.

Then, 125 ml of ethyl acetate was added to 27.5 g (0.11 mol) of copper(II) bromide, followed by heating under reflux, and then a solution of 18.0 g (0.05 mol) of the 4'-hexadecyloxyacetophenone obtained above in 60 ml of chloroform was added thereto, dropwise, over 1 hour. After the addition, the mixture was refluxed for 1.5 hours. The temperature was brought to room temperature, the deposited crystals of copper(I) bromide were filtered off, and the filtrate was washed with water and dried over magnesium sulfate. After the solvent was removed by distillation, crystallization from methanol was carried out, to obtain 20.0 g of colorless crystals of 4'-hexadecyloxyphenacyl bromide. Yield: 91%.

Then, 4.39 g (0.01 mol) of the 4'-hexadecyloxyphenacyl bromide and 1.80 g (0.01 mol) of theophylline were dispersed in 30 ml of acetonitrile, and then 1.6 ml of triethylamine was added to the dispersion, followed by stirring for 20 hours at room temperture. Water was added to the reaction mixture, and the deposited crystals were filtered, to obtain 4.24 g of pale yellow crystals of 4'-hexadecyloxybenzoylmethyltheophylline. Yield: 79%. Melting point: 109 to 112°C.

0.26 g (6.6 mmol) of sodium hydride (60%) was dispersed in 20 ml of N,N-dimethylformamide, and then 0.82 g (3 mmol) of (I)-24 and 1.62 g (3 mmol) of the 4'-hexadecyloxybenzoylmethyltheophylline were added to the dispersion under a nitrogen atmosphere, followed by stirring for 3 hours at room temperature.

Water and ethyl acetate were added to the reaction mixture; the organic layer was separated and washed with water; the solvent was distilled off, and the residue was purified by silica gel column chromatography (n-hexane/ethyl acetate = from 1/1 to 1/2) and was crystallized from n-hexane, to obtain 1.20 g of (IV)-87. Yield: 56%. Melting point: 109-111°C.

### Example 9 (Synthesis of (IV)-93 by Using (I)-19)

3.0 g (0.022 mol) of α-chloropinacolone and 6.8 g (0.02 mol) of 4-(4-benzyloxyphenylsulfonyl)phenol were dispersed in 50 ml of N,N-dimethylformamide, and then 3.1 g (0.022 mol) of potassium carbonate was added to the dispersion, followed by heating on a steam bath for 2 hours with stirring. The temperature was brought to room temperature; 200 ml of ethyl acetate was added thereto; the organic layer was washed with water; then the solvent was removed by distillation, and the residue was crystallized from acetonitrile, to obtain 8.7 g of colorless crystals of 4-(4-benzyloxyphenylsulfonyl)phenoxypinacolone. Melting point: 195-197°C. Yield: 98%.

Then, 0.46 g (11 mmol) of sodium hydride (60%) was added to 20 ml of N,N-dimethylformamide, and then a solution of 1.65 g (3 mmol) of (I)-19 and 2.0 g (4.5 mmol) of the 4-(4-benzyloxyphenylsulfonyl)phenoxypinacolone obtained above, in 15 ml of N,N-dimethylformamide, was added thereto under a nitrogen atmosphere over 15 min. The reaction mixture was stirred at room temperature for 3 hours; then it was poured into ice water and neutralized with hydrochloric acid, followed by extraction with ethyl acetate. After the solvent was removed by distillation, the residue was purified by silica gel column chromatography (n-hexane/ethyl acetate = 3/1) and was crystallized from n-hexane/ethyl acetate mixed solvent, to obtain 1.30 g of colorless crystals of (IV)-93. Yield: 48%. Melting point: 109-111°C.

### Example 10

In a similar manner, the synthesis was carried out. The melting points of typical examples of the compound represented by formula (IV) are shown below.

**Table 4**

| Compound | Melting point (°C) |
|---|---|
| (IV)-81 | 77 - 79 |
| (IV)-82 | 134 - 135 |
| (IV)-83 | 78 - 80 |
| (IV)-84 | 124 - 126 |
| (IV)-85 | 83 - 86 |
| (IV)-90 | 95 - 103 |
| (IV)-91 | 120 - 122 |
| (IV)-93 | 109 - 111 |
| (IV)-94 | 94 - 98 |
| (IV)-95 | 135 - 138 |

### Example 11 Synthesis of (IX)-9

First, using the method described in the literature, 2-phenylcarbamoylimidazole was synthesized (J. Org. Chew., 42, 3925 (1977)).

That is, 13.6 g (0.2 mol) of imidazole and 23.8 g (0.2 mol) of phenylisocyanate were added to 50 ml of o-nitrotoluene, followed by heating on an oil bath at 230°C for 1.5 hours with stirring. The temperature was brought to room temperature; then 200 ml of carbon tetrachloride was added thereto, and the deposited crystals were filtered, to obtain 26.0 g of crude crystals of 2-phenylcarbamoylimidazole. The crude crystals were dispersed in 100 ml of acetonitrile, and then the dispersion was filtered, to obtain 18.5 g of purified 2-phenylcarbamoylimidazole. Yield: 50%.

Then, 3.74 g (0.02 mol) of 2-phenylcarbamoylimidazole and 3.0 g (0.022 mol) of chloropinacolone were dispersed in 50 ml of acetonitrile, and then 3.1 ml of triethylamine was added to the dispersion, followed by heating for 15 hours under reflux. The temperature was brought to room temperature; ethyl acetate and water were added thereto; the organic layer was separated and washed with water, and the solvent was removed by distillation.

20 ml of acetonitrile was added thereto, followed by heating for 20 min under reflux; then the temperature was brought to room temperature, the deposited crystals (recovered 2-phenylcarbamoylimidazole) were filtered (1.0 g) off, and the filtrate was condensed and was purified by silica gel column chromatography (n-hexane/ethyl acetate = 2/1), followed by crystallization from n-hexane, to obtain 3.2 g of Compound (IX)-9. Yield: 56%.
- Melting point:: 141-143°C
- ¹H-NMR: (300 MHz; CDCl₃)
- δppm: 1.31 (s, 9H)
5.59 (s, 2H)
6.95 (s, 1H)
7.12 (m, 2H)
7.32 (m, 2H)
7.60 (d, 2H, J = 8.0 Hz)
9.23 (bs, 1H)

### Example 12 Synthesis of (IX)-12

(IX)-12 was synthesized in the same way as in Example 11.
- Melting point:: 178-180°C
- ¹H-NMR: (200 MHz; CDCl₃)
- δppm: 3.89 (s, 3H)
6.02 (s, 2H)
6.98 (d, 2H, J = 8.7 Hz)
7.05-7.4 (m, 5H)
7.60 (d, 2H, J = 8.0 Hz)
8.02 (d, 2H, J = 8.7 Hz)
9.28 (bs, 1H)

### Example 13 Synthesis of (IX)-15

(IX)-15 was synthesized in the same way as in Example 11.
- Melting point:: 120-123°C
- ¹H-NMR: (200 MHz; CDCl₃)
- δppm: 0.89 (t, 3H, J = 6.3 Hz)
1.27 (m, 26H)
1.82 (m, 2H)
4.05 (t, 2H, J = 6.3 Hz)
6.03 (s, 2H)
6.97 (d, 2H, J = 10.0 Hz)
7.1-7.4 (m, 5H)
7.59 (d, 2H, J = 8.0 Hz)
8.00 (d, 2H, J = 10.0 Hz)
9.30 (bs, 1H)

### Example 14 Synthesis of (IX)-22

2.29 g (0.01 mol) of p-methoxyphenacyl bromide and 1.80 g (0.01 mol) of theophylline were dispersed in 30 ml of acetonitrile, and then 1.6 ml of triethylamine was added to the dispersion, followed by stirring at room temperature for 20 hours. 50 ml of water was added thereto, and the deposited crystals were filtered, to obtain 2.8 g of colorless crystals of (IX)-22. Yield: 85%.
- Melting point:: 201-203°C
- ¹H-NMR: (200 MHz; CDCl₃)
- δppm: 3.37 (s, 3H)
3.64 (s, 3H)
3.93 (s, 3H)
5.79 (s, 2H)
7.00 (d, 2H, J = 9.0 Hz)
7.66 (s, 1H)
8.01 (d, 2H, J = 9.0 Hz)

### Example 15 Synthesis of (IX)-24

27.2 g (0.2 mol) of p-hydroxyacetophenone and 61.0 g (0.2 mol) of hexadecyl bromide were dissolved in 300 ml of N,N-dimethylformamide, and then 27.8 g (0.2 mol) of potassium carbonate was added to the solution, followed by stirring for 4 hours, with the internal temperature being 80 to 90°C. The temperature was brought to room temperature; then 500 ml of water was added thereto, and the deposited crystals were filtered, to obtain 70.0 g of p-hexadecyloxyacetophenone. Yield: 97%.

27.5 g (0.11 mol) of copper(II) bromide was dispersed in 125 ml of ethyl acetate, and while the dispersion was heated under reflux, a solution of 18.0 g (0.05 mol) of the p-hexadecyloxyacetophenone obtained above, in 60 ml of chloroform, was added, dropwise, to the dispersion over 30 min. After the addition, the reaction mixture was heated for 2 hours under reflux; then the temperature was brought to room temperature; the deposited crystals of copper(I) bromide were filtered off; ethyl acetate was added to the filtrate, and the organic layer was washed with water twice. The organic layer was dried over magnesium sulfate, the solvent was removed by distillation, and crystallization from methanol was carried out, to obtain 20.0 g of p-hexadecyloxy-α-bromoacetophenone.

Then, 1.80 g (0.01 mol) of theophylline was added to 4.39 g (0.01 mol) of the p-hexadecyloxy-α-bromoacetophenone, and then 30 ml of acetonitrile and 1.6 ml of triethylamine were added thereto, followed by stirring at room temperature for 20 hours.

Then water was added thereto, and the deposited crystals were filtered, to obtain 4.24 g of Compound (IX)-24. Yield: 79%.
- Melting point:: 109-112°C
- ¹H-NMR: (200 MHz; CDCl₃)
- δppm: 0.88 (t, 3H, J = 6.7 Hz)
1.28 (m, 26H)
1.80 (m, 2H)
3.35 (s, 3H)
3.63 (s, 3H)
4.04 (t, 2H, J = 6.7 Hz)
5.75 (s, 2H)
6.96 (d, 2H, J = 9.3 Hz)
7.65 (s, 1H)
7.98 (d, 2H, J = 9.3 Hz)

### Example 16 Synthesis of (IX)-28 or (IX)-29

4-methoxycarbonylimidazole was synthesized in accordance with the method described in the literature (Synthesis, 1975, 162).

Then, 1.10 g (8.7 mmol) of the 4-methoxycarbonylimidazole and 1.20 g (8.7 mmol) of α-chloropinacolone were dissolved in 30 ml of N,N-dimethylformamide, and then 1.32 g (9.5 mmol) of potassium carbonate was added to the solution, followed by stirring under a nitrogen atmosphere at room temperature for 20 hours.

Then 50 ml of ethyl acetate and 100 ml of water were added thereto, the organic layer was separated and washed with water twice, and the solvent was removed by distillation.

30 ml of n-hexane and 15 ml of ethyl acetate were added to the thus obtained oil, and the deposited crystals were filtered, to obtain the desired product (IX)-28 or (IX)-29 as a single product.
- Melting point:: 153-155°C
- ¹H-NMR: (300 MHz; CDCl₃)
- δppm: 1.26 (s, 9H), 3.89 (s, 3H), 4.96 (s, 2H), 7.45 (s, 1H), 7.53 (s, 1H)

Typical examples of the yellow coupler synthesized from the compound of the second invention are shown below:

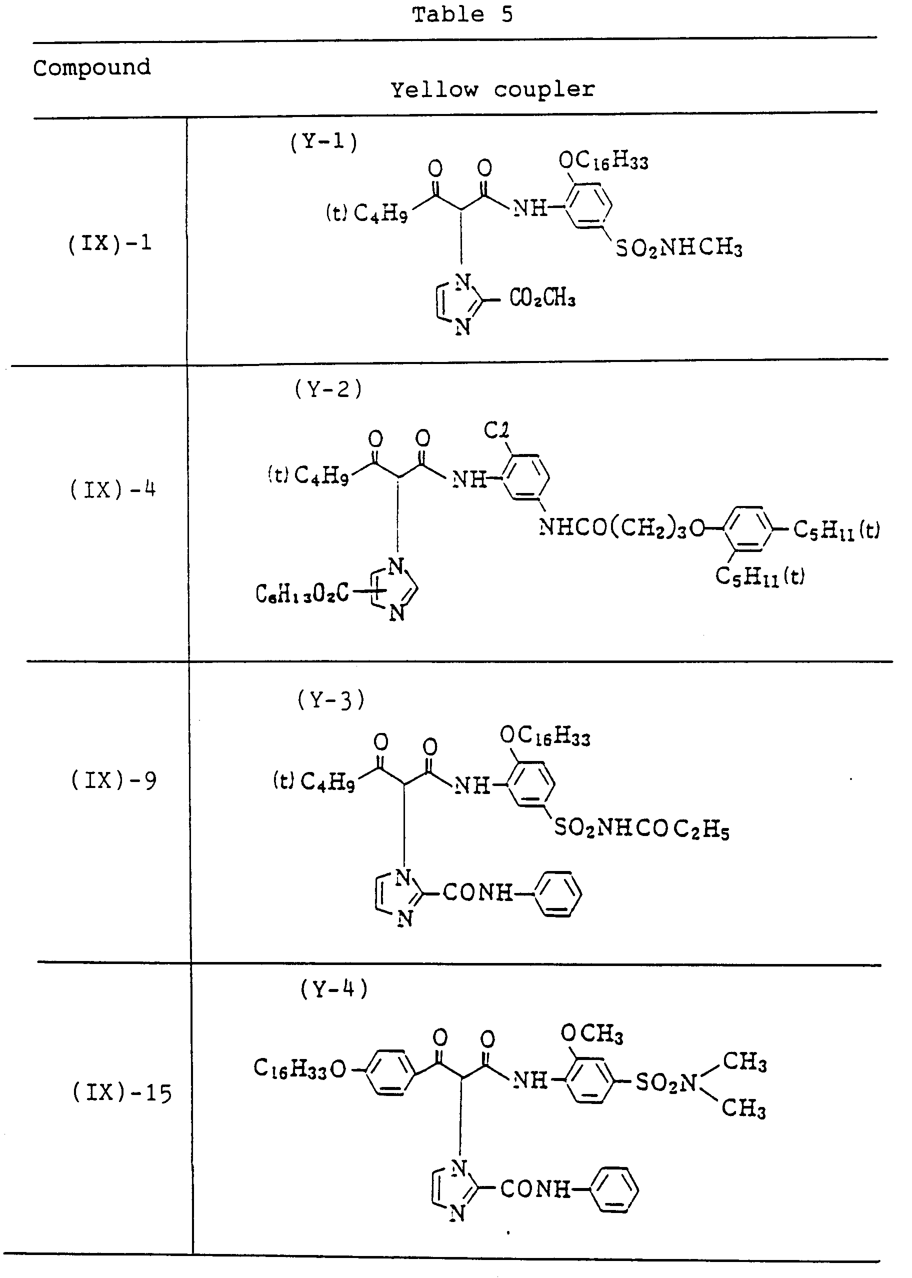

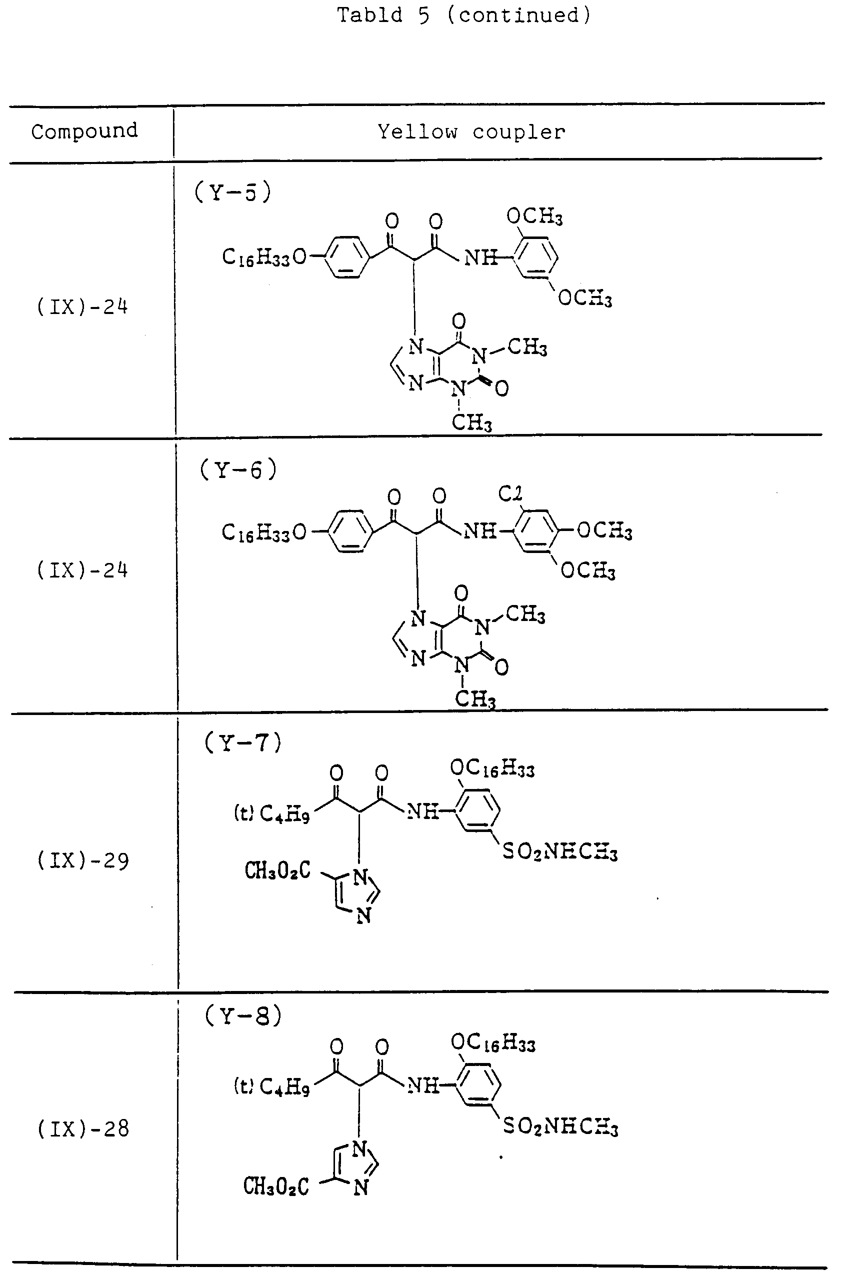

The melting points of typical examples of the yellow coupler are:
- (Y-3):: 116-118°C
- (Y-4):: 83-86°C
- (Y-5):: 109-111°C

Thus, the compound of the second invention is a synthetic intermediate useful as an imidazole-coupling-off-type two-equivalent yellow coupler.

### Example 17 Synthesis of (IV)-87 (Yellow Coupler (Y-5)) by Using a Compound (IX)-24

15.3 g (0.1 mol) of 2,5-dimethoxyaniline was dispersed in 100 ml of acetonitrile, and then 13.4 ml of phenyl chlorocarbonate was added, dropwise, to the dispersion, over 10 min under cooling with ice. After this was stirred for 30 min, 8.3 ml of pyridine was added, dropwise, thereto over 10 min.

The reaction was continued for 1 hour; then ethyl acetate and water were added thereto, the organic layer was separated and washed with water, and the solvent was removed by distillation. 200 ml of methylene chloride and 50 g of silica gel were added to the residue, followed by stirring, to allow the colored components to be adsorbed to the silica gel, and also to cause elution with methylene chloride to occur; then condensation was carried out, and then crystallization with n-hexane was carried out, to obtain 24.3 g of N-(2,5-dimethoxyphenyl)phenoxycarbonamide. Yield: 89%. Melting point: 92-94°C.

Then, 0.26 g (6.6 mmol) of sodium hydride (60%) was dispersed in 20 ml of N,N-dimethylformamide, and then 1.62 g (3 mmol) of (IX)-24, which is a compound of the second invention, and 0.82 g of the thus synthesized N-(2,5-dimethoxyphenyl)phenoxycarbonamide, were added to the dispersion under a nitrogen atmosphere, followed by stirring at room temperature for 3 hours.

Then water and ethyl acetate were added to the reaction mixture; the organic layer was separated and washed with water; the solvent was removed by distillation, and the residue was purified by silica gel column chromatography (n-hexane/ethyl acetate = from 1/1 to 1/2), followed by crystallization with n-hexane, to obtain 1.20 g of (IV)-87 (Yellow Coupler (Y-5)). Yield: 56%.
- Melting point:: 109-111°C
- ¹H-NMR: (200 MHz; CDCl₃)
- δppm: 0.88 (t, 3H, J = 6.3 Hz)
1.28 (m, 26H)
1.82 (m, 2H)
3.46 (s, 3H)
3.63 (s, 3H)
3.70 (s, 3H)
3.72 (s, 3H)
4.04 (t, 2H, J = 6.3 Hz)
6.57 (dd, 1H, J = 8.0, 3.3 Hz)
6.75 (d, 1H, J = 8.0 Hz)
7.02 (d, 2H, J = 8.7 Hz)
7.77 (s, 1H)
7.90 (d, 1H, J = 3.3 Hz)
8.12 (d, 2H, J = 8.7 Hz)
8.40 (s, 1H)
8.60 (s, 1H)

The starting material, R¹⁷-H, of the acetyl compound of the second invention of the present invention can be obtained easily and inexpensively, and therefore a two-equivalent yellow coupler can be provided at low cost. Further, (IV)-87, which is a typical example of the yellow coupler that can be derived from the acetyl compound of the second invention, is known to be a highly active two-equivalent yellow coupler (U.S. Patent No. 4,032,347). (IV)-87 is particularly useful to lower cost as a yellow coupler for color photographic materials, in particular for color papers, because, for example, (IV)-87 can reduce the amount of a silver halide to be used.

## Claims

1. A method for producing a compound represented by the following formula (IV): 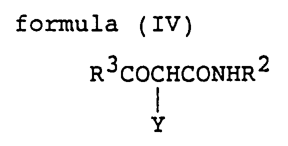 wherein R² represents an alkyl group, a cycloalkyl group, or an aryl group; R³ represents an alkyl group bonded through a secondary or tertiary carbon atom, a cycloalkyl group, a cyclic ether group, an aryl group, a group represented by formula (III), an alkoxy group, or an N,N-di-substituted amino group, in which N,N-di-substituted amino group the substituents may bond together to form a nitrogen-containing heterocyclic ring together with the nitrogen atom; and Y represents a hydrogen atom or an electron-attracting group: 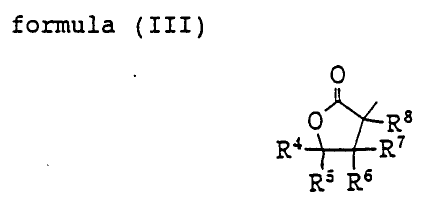 wherein R⁴, R⁵, R⁶, R⁷, and R⁸ each represent a hydrogen atom, an alkyl group, a benzyl group, an aryl group, an alkoxy group, an aryloxy group, an alkylthio group, or an arylthio group, comprising reacting a compound represented by formula (I):
R¹OCONHR² formula (I)
wherein R¹ represents an aryl group and R² has the same meaning as defined above, with a compound represented by formula (II):
R³COCH₂Y formula (II)
wherein R³ and Y each have the same meaning as defined above, in the presence of a base.

2. The method for producing a compound represented by formula (IV) as claimed in claim 1, wherein R¹ in the compound represented by formula (I) is a phenyl group, and wherein R² is a phenyl group that may be substituted by a group selected from a halogen atom, an alkoxycarbonyl group, an acylamino group, an alkylsulfamoyl group, an arylsulfamoyl group, an alkylsulfonamide group, an alkoxy group, a nitro group, a cyano group, and an acylsulfamoyl group.

3. The method for producing a compound represented by formula (IV) as claimed in claim 1 or 2, wherein Y in the compound represented by formula (II) is a hydrogen atom, a cyano group, an alkylcarbonyl group having 2 to 18 carbon atoms, an arylcarbonyl group, a nitro group, an alkoxycarbonyl group having 2 to 18 carbon atoms, a carbamoyl group, a substituted carbamoyl group having 2 to 18 carbon atoms, an alkylsulfonyl group having 1 to 18 carbon atoms, an arylsulfonyl group having 6 to 20 carbon atoms, an aryloxy group having 6 to 20 carbon atoms, or a heterocyclic group.

4. The method for producing a compound represented by formula (IV) as claimed in claim 1 or 2, wherein Y in the compound represented by formula (II) is selected from the group consisting of a hydrogen atom, a cyano group, an alkylcarbonyl group having 2 to 18 carbon atoms, an alkoxycarbonyl group having 2 to 18 carbon atoms, an aryloxy group having 6 to 20 carbon atoms, imido-type 5-membered heterocyclic rings represented by the following formula (V), (VI), or (VII), and a 5-membered heterocyclic ring bonded through a nitrogen atom represented by the following formula (VIII): 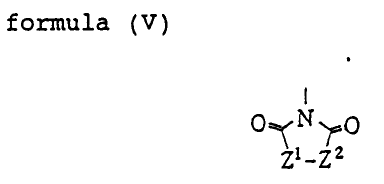 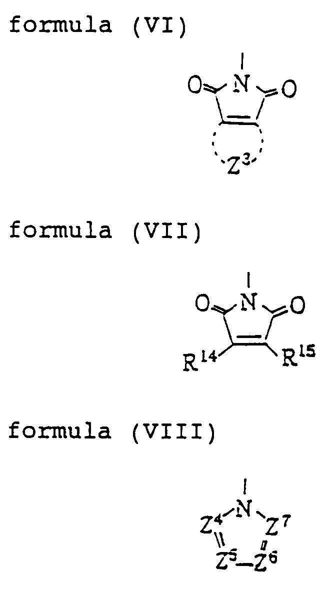 wherein Z¹ and Z² each represent an oxygen atom, a group represented by -N(R¹¹)-, or a group represented by -(R¹²)C(R¹³)-, wherein R¹¹ and R¹² each represents a hydrogen atom, an alkyl group, a benzyl group, or an aryl group, and R¹³ represents a hydrogen atom, an alkyl group, a benzyl group, an aryl group, or an alkoxy group; Z³ represents a group of nonmetal atoms required to form a 5- to 7-membered ring; R¹⁴ and R¹⁵ each represent a hydrogen atom, an alkyl group, or an aryl group; and Z⁴, Z⁵, Z⁶, and Z⁷ each represent a nitrogen atom or a substituted or an unsubstituted methine group, and Z⁴ and Z⁵, Z⁵ and Z⁶, or Z⁶ and Z⁷ may bond together to form a 5- to 7-membered ring, provided that each of Z⁴, Z⁵, Z⁶, and Z⁷ in formula (VIII) does not represent a nitrogen atom at the same time.

5. The method for producing a compound represented by formula (IV) as claimed in claim 1 or 2, wherein, Y in the compound represented by formula (II) is a cyano group, an alkylcarbonyl group having 2 to 18 carbon atoms, or an alkoxycarbonyl group having 2 to 18 carbon atoms, and R³ is an indoline-1-yl group.

6. The method for producing a compound represented by formula (IV) as claimed in claim 1, 2, 3, 4, or 5, wherein the base is a sodium hydride.

7. A substituted acetyl compound represented by formula (IX):
R¹⁶ - COCH₂R¹⁷ formula (IX)
wherein R¹⁶ represents a tert-butyl group or a phenyl group having an alkoxy group with 1 to 18 carbon atoms in the paraposition, and wherein R¹⁷ represents a group represented by formula (X) or formula (XI): 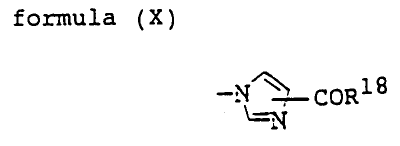 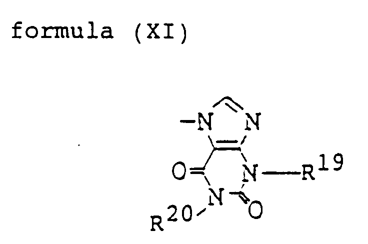 wherein R¹⁸ represents an alkoxy group having 1 to 18 carbon atoms or an anilino group, and R¹⁹ and R²⁰ each represent an alkyl group having 1 to 18 carbon atoms.

8. The substituted acetyl compound as claimed in claim 7, wherein in formula (IX) the 1-imidazolyl group represented by formula (X) has a group -COR¹⁸ in the 2-position and R¹⁸ represents an anilino group.

9. The substituted acetyl compound as claimed in claim 8, wherein in formula (IX) the 1-imidazolyl group represented by formula (X) has a group -COR¹⁸ in the 4- or 5-position and R¹⁸ represents an alkoxy group having 1 to 18 carbon atoms.

## Patentansprüche

1. Verfahren zur Herstellung einer Verbindung mit der folgenden Formel (IV) 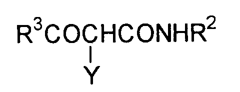 worin R² eine Alkylgruppe, eine Cycloalkylgruppe oder eine Arylgruppe darstellt; R³ eine Alkylgruppe, die über ein sekundäres oder tertiäres Kohlenstoffatom gebunden ist, eine Cycloalkylgruppe, eine cyclische Ethergruppe, eine Arylgruppe, eine Gruppe mit der Formel (III), eine Alkoxygruppe oder eine N,N-disubstituierte Aminogruppe, bei der die Substituenten miteinander unter Bildung eines stickstoffhaltigen heterocyclischen Rings zusammen mit dem Stickstoffatom verbunden sein können, darstellt, und Y ein Wasserstoffatom oder eine elektronenziehende Gruppe darstellt, 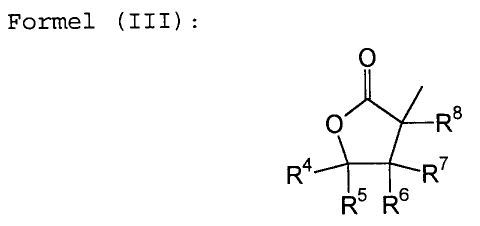 worin R⁴, R⁵, R⁶, R⁷ und R⁸ jeweils ein Wasserstoffatom, eine Alkylgruppe, eine Benzylgruppe, eine Arylgruppe, eine Alkoxygruppe, eine Aryloxygruppe, eine Alkylthiogruppe oder eine Arylthiogruppe bedeuten, umfassend die Umsetzung einer Verbindung mit der Formel (I):
R¹OCONHR²
worin R¹ eine Arylgruppe bedeutet und R² die gleiche Bedeutung wie oben hat, mit einer Verbindung der Formel (II):
R³COCH₂Y
worin R³ und Y jeweils die gleichen Bedeutungen wie oben haben, in Gegenwart einer Base.

2. Verfahren zur Herstellung einer Verbindung mit der Formel (IV) gemäss Anspruch 1, worin R¹ in der Verbindung mit der Formel (I) eine Phenylgruppe ist und worin R² eine Phenylgruppe ist, die mit einer Gruppe, ausgewählt aus einem Halogenatom, einer Alkoxycarbonylgruppe, einer Acylaminogruppe, einer Alkylsulfamoylgruppe, einer Arylsulfamoylgruppe, einer Alkylsulfonamidgruppe, einer Alkoxygruppe, einer Nitrogruppe, einer Cyanogruppe und einer Acylsulfamoylgruppe substituiert sein kann.

3. Verfahren zur Herstellung einer Verbindung mit der Formel (IV) gemäss Anspruch 1 oder 2, worin Y in der Verbindung mit der Formel (II) ein Wasserstoffatom, eine Cyanogruppe, eine Alkylcarbonylgruppe mit 2 bis 18 Kohlenstoffatomen, eine Arylcarbonylgruppe, eine Nitrogruppe, eine Alkoxycarbonylgruppe mit 2 bis 18 Kohlenstoffatomen, eine Carbamoylgruppe, eine substituierte Carbamoylgruppe mit 2 bis 18 Kohlenstoffatomen, eine Alkylsulfonylgruppe mit 1 bis 18 Kohlenstoffatomen, eine Arylsulfonylgruppe mit 6 bis 20 Kohlenstoffatomen, eine Aryloxygruppe mit 6 bis 20 Kohlenstoffatomen oder eine heterocyclische Gruppe ist.

4. Verfahren zur Herstellung einer Verbindung mit der Formel (IV) gemäss Anspruch 1 oder 2, worin Y in der Verbindung mit der Formel (II) ausgewählt wird aus einem Wasserstoffatom, einer Cyanogruppe, einer Alkylcarbonylgruppe mit 2 bis 18 Kohlenstoffatomen, einer Alkoxycarbonylgruppe mit 2 bis 18 Kohlenstoffatomen, einer Aryloxygruppe mit 6 bis 20 Kohlenstoffatomen, 5-gliedrigen heterocyclischen Imidringen mit den folgenden Formeln (V), (VI) oder (VII), und einem 5-gliedrigen heterocyclischen Ring, der über ein Stickstoffatom verbunden ist und durch die folgende Formel (VIII) dargestellt wird: 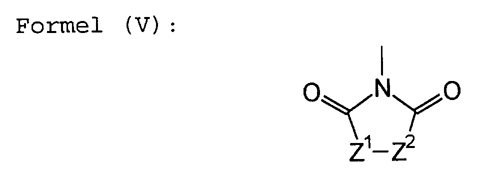 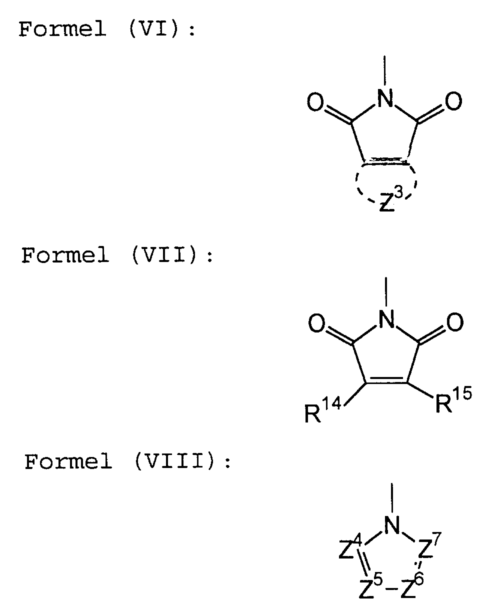 worin Z¹ und Z² jeweils ein Sauerstoffatom, eine Gruppe -N(R¹¹)- oder eine Gruppe -(R¹²)C(R¹³)-bedeuten, worin R¹¹ und R¹² jeweils ein Wasserstoffatom, eine Alkylgruppe, eine Benzylgruppe oder eine Arylgruppe bedeuten und R¹³ ein Wasserstoffatom, eine Alkylgruppe, eine Benzylgruppe, eine Arylgruppe oder eine Alkoxygruppe darstellt; Z³ eine Gruppe aus nicht-metallischen Atomen bedeutet, die zur Bildung eines 5- bis 7-gliedrigen Rings erforderlich ist; R¹⁴ und R¹⁵ jeweils ein Wasserstoffatom, eine Alkylgruppe oder eine Arylgruppe bedeuten; und Z⁴, Z⁵, Z⁶ und Z⁷ jeweils ein Stickstoffatom oder eine substituierte oder unsubstituierte Methingruppe bedeuten, und Z⁴ und Z⁵, Z⁵ und Z⁶ oder Z⁶ und Z⁷ miteinander unter Bildung eines 5- bis 7-gliedrigen Rings verbunden sein können, mit der Massgabe, dass nicht alle Gruppen Z⁴, Z⁵, Z⁶ und Z⁷ in Formel (VIII) gleichzeitig ein Stickstoffatom bedeuten.

5. Verfahren zur Herstellung einer Verbindung mit der Formel (IV) gemäss Anspruch 1 oder 2, worin Y in der Verbindung mit der Formel (II) eine Cyanogruppe, eine Alkylcarbonylgruppe mit 2 bis 18 Kohlenstoffatomen oder eine Alkoxycarbonylgruppe mit 2 bis 18 Kohlenstoffatomen ist, und R³ eine Indolin-1-yl-Gruppe ist.

6. Verfahren zur Herstellung einer Verbindung der Formel (IV) gemäss Anspruch 1, 2, 3, 4 oder 5, wobei die Base Natriumhydrid ist.

7. Substituierte Acetylverbindung mit der Formel (IX)
R¹⁶―COCH₂R¹⁷
worin R¹⁶ eine tert-Butylgruppe oder eine Phenylgruppe mit einer Alkoxygruppe mit 1 bis 18 Kohlenstoffatomen in para-Stellung bedeutet, und R¹⁷ eine Gruppe mit der Formel (X) oder (XI) bedeutet, 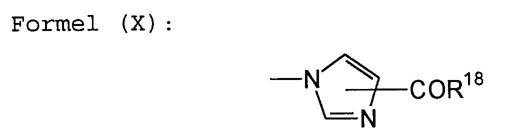 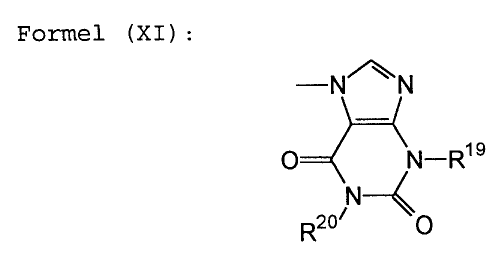 worin R¹⁸ eine Alkoxygruppe mit 1 bis 18 Kohlenstoffatomen oder eine Anilinogruppe bedeutet und R¹⁹ und R²⁰ jeweils eine Alkylgruppe mit 1 bis 18 Kohlenstoffatomen bedeuten.

8. Substituierte Acetylverbindung gemäss Anspruch 7, wobei in Formel (IX) die 1-Imidazolylgruppe mit der Formel (X) eine Gruppe -COR¹⁸ in 2-Position hat und R¹⁸ eine Anilinogruppe bedeutet.

9. Substituierte Acetylverbindung gemäss Anspruch 8, worin in Formel (IX) die 1-Imidazolylgruppe mit der Formel (X) eine Gruppe -COR¹⁸ in 4- oder 5-Position aufweist, und R¹⁸ eine Alkoxygruppe mit 1 bis 18 Kohlenstoffatomen bedeutet.

## Revendications

1. Procédé pour la production d'un composé représenté par la formule (IV) ci-dessous: 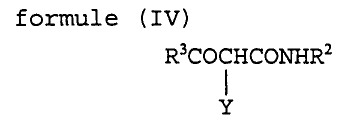 dans laquelle R² représente un groupe alkyle, un groupe cycloalkyle ou un groupe aryle; R³ représente un groupe alkyle lié par l'intermédiaire d'un atome de carbone secondaire ou tertiaire, un groupe cycloalkyle, un groupe éther cyclique, un groupe aryle, un groupe représenté par la formule (III), un groupe alcoxy ou un groupe amino N,N-disubstitué, dans lequel groupe amino N,N-disubstitué, les substituants peuvent être liés ensemble pour former, avec l'atome d'azote, un cycle hétérocyclique contenant de l'azote; et Y représente un atome d'hydrogène ou un groupe attirant les électrons: 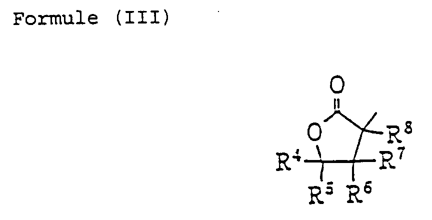 dans laquelle R⁴, R⁵, R⁶, R⁷ et R⁸ représentent chacun un atome d'hydrogène, un groupe alkyle, un groupe benzyle, un groupe aryle, un groupe alcoxy, un groupe aryloxy, un groupe alkylthio ou un groupe arylthio, qui comporte la mise en réaction d'un composé représenté par la formule (I):
R¹OCONHR² formule (I)
dans laquelle R¹ représente un groupe aryle et R² a la même signification que celle donnée plus haut, avec un composé représenté par la formule (II):
R³COCH₂Y formule (II)
dans laquelle R³ et Y ont chacun la même signification que celle donnée plus haut, en présence d'une base.

2. Procédé pour la production d'un composé représenté par la formule (IV) selon la revendication 1, dans lequel, dans le composé représenté par la formule (I), R¹ est un groupe phényle, et dans lequel R² est un groupe phényle qui peut être substitué avec un groupe choisi parmi un atome d'halogène, un groupe alcoxycarbonyle, un groupe acylamino, un groupe alkylsulfamoyle, un groupe arylsulfamoyle, un groupe alkylsulfonamide, un groupe alcoxy, un groupe nitro, un groupe cyano et un groupe acylsulfamoyle.

3. Procédé pour la production d'un composé représenté par la formule (IV) selon la revendication 1 ou 2, dans lequel, dans le composé représenté par la formule (II), Y est un atome d'hydrogène, un groupe cyano, un groupe alkylcarbonyle comptant de 2 à 18 atomes de carbone, un groupe arylcarbonyle, un groupe nitro, un groupe alcoxycarbonyle comptant de 2 à 18 atomes de carbone, un groupe carbamoyle, un groupe carbamoyle substitué comptant de 2 à 18 atomes de carbone, un groupe alkylsulfonyle comptant de 1 à 18 atomes de carbone, un groupe arylsulfonyle comptant de 6 à 20 atomes de carbone, un groupe aryloxy comptant de 6 à 20 atomes de carbone ou un groupe hétérocyclique.

4. Procédé pour la production d'un composé représenté par la formule (IV) selon la revendication 1 ou 2, dans lequel, dans le composé représenté par la formule (II), Y est choisi dans le groupe constitué d'un atome d'hydrogène, d'un groupe cyano, d'un groupe alkylcarbonyle comptant de 2 à 18 atomes de carbone, d'un groupe alcoxycarbonyle comptant de 2 à 18 atomes de carbone, d'un groupe aryloxy comptant de 6 à 20 atomes de carbone, de cycles hétérocycliques à 5 chaînons de type imido, représentés par la formule (V), (VI) ou (VII) ci-dessous, et d'un cycle hétérocyclique à 5 chaînons, lié par l'intermédiaire d'un atome d'azote, représenté par la formule (VIII) ci-dessous: 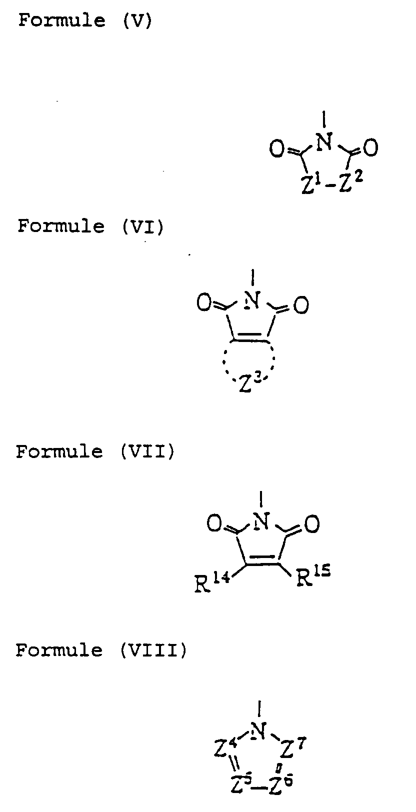 dans lesquelles Z¹ et Z² représentent chacun un atome d'oxygène, un groupe représenté par -N(R¹¹)- ou un groupe représenté par -(R¹²)C(R¹³)-, R¹¹ et R¹² représentant chacun un atome d'hydrogène, un groupe alkyle, un groupe benzyle ou un groupe aryle, R¹³ représente un atome d'hydrogène, un groupe alkyle, un groupe benzyle, un groupe aryle ou un groupe alcoxy; Z³ représente un groupe d'atomes de non métaux nécessaire pour former un cycle comptant de 5 à 7 chaînons; R¹⁴ et R¹⁵ représentent chacun un atome d'hydrogène, un groupe alkyle ou un groupe aryle; et Z⁴, Z⁵, Z⁶ et Z⁷ représentent chacun un atome d'azote ou un groupe méthine substitué ou non substitué, et Z⁴ et Z⁵, Z⁵ et Z⁶ ou Z⁶ et Z⁷ peuvent être liés ensemble pour former un cycle comptant de 5 à 7 chaînons, pour autant que chacun parmi Z⁴, Z⁵, Z⁶ et Z⁷ de la formule (VIII) ne représente pas simultanément un atome d'azote.

5. Procédé pour la production d'un composé représenté par la formule (IV) selon la revendication 1 ou 2, dans lequel, dans le composé représenté par la formule (II), Y est un groupe cyano, un groupe alkylcarbonyle comptant de 2 à 18 atomes de carbone ou un groupe alcoxycarbonyle comptant de 2 à 18 atomes de carbone, et R³ est un groupe indoline-1-yle.

6. Procédé pour la production d'un composé représenté par la formule (IV) selon la revendication 1, 2, 3, 4 ou 5, dans lequel la base est un hydrure de sodium.

7. Composé acétyle substitué représenté par la formule (IX):
R¹⁶-COCH₂R¹⁷ formule (IX)
dans laquelle R¹⁶ représente un groupe tert-butyle ou un groupe phényle présentant un groupe alcoxy comptant de 1 à 18 atomes de carbone en position para, et dans lequel R¹⁷ représente un groupe représenté par la formule (X) ou la formule (XI) : 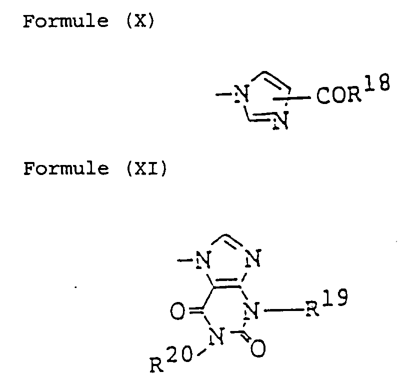 dans lesquelles R¹⁸ représente un groupe alcoxy comptant de 1 à 18 atomes de carbone ou un groupe anilino, et R¹⁹ et R²⁰ représentent chacun un groupe alkyle comptant de 1 à 18 atomes de carbone.

8. Composé acétyle substitué selon la revendication 7, dans lequel, dans la formule (IX), le groupe 1-imidazolyle représenté par la formule (X) présente un groupe -COR¹⁸ en position 2, et R¹⁸ représente un groupe anilino.

9. Composé acétyle substitué selon la revendication 8, dans lequel, dans la formule (IX), le groupe 1-imidazolyle représenté par la formule (X) présente un groupe -COR¹⁸ en position 4 ou en position 5, et R¹⁸ représente un groupe alcoxy comptant de 1 à 18 atomes de carbone.
